# EUROPÄISCHE PATENTSCHRIFT

(11) **EP 2 736 585 B1**
(45) Veröffentlichungstag und Bekanntmachung des Hinweises auf die Patenterteilung: **15.05.2019**
(21) Anmeldenummer: 12743677.2
(22) Anmeldetag: 27.07.2012
(51) Int. Cl.: A61M 39/22, A61M 39/02, A61M 5/14, A61M 5/168, F16K 11/085

(54) **MEDIZINISCHER PORT, BLUTSCHLAUCH ZUR VERWENDUNG BEI EINER EXTRAKORPORALEN BLUTBEHANDLUNG SOWIE MEDIZINISCHE BEHANDLUNGSVORRICHTUNG**
MEDICAL PORT, BLOOD LINE FOR USE DURING AN EXTRACORPOREAL BLOOD TREATMENT, AND MEDICAL TREATMENT DEVICE
RACCORD MÉDICAL, TUBULURE SANGUINE DESTINÉE À ÊTRE UTILISÉE DANS UN TRAITEMENT EXTRACORPOREL DU SANG, AINSI QUE DISPOSITIF DE TRAITEMENT MÉDICAL

(30) Priorität: 29.07.2011 DE 102011108787; 29.07.2011 US 201161512946 P
(43) Veröffentlichungstag der Anmeldung: 04.06.2014
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2012/003217
(87) Internationale Veröffentlichungsnummer: WO 2013/017247

(56) Entgegenhaltungen:
- EP-A1- 1 234 596
- EP-A1- 1 555 041
- WO-A2-2011/143049
- US-A- 4 230 128
- US-A- 5 135 026
- US-A1- 2010 319 796

## Beschreibung

Die vorliegende Erfindung betrifft einen medizinischen Port gemäß Anspruch 1. Sie betrifft zudem einen Blutschlauch gemäß Anspruch 12 und eine medizinische Behandlungsvorrichtung gemäß Anspruch 13.

Beim Einsatz von medizinischen Behandlungsvorrichtungen kommen ebenso wie bei Analysegeräten des Laborbereichs vielfach auch Fluidsysteme mit Schläuchen, Konnektoren, Beuteln, Pumpen, Kammern usw. zum Einsatz.

Dabei wird regelmäßig einem durch einen Hauptkanal des Fluidsystems strömenden ersten Fluid, meist eine Flüssigkeit, beispielsweise Blut, Dialyseflüssigkeit oder Infusionslösung, eine Probe entnommen. Ebenso ist es üblich, dem ersten Fluid ein zweites Fluid, beispielsweise eine Behandlungsflüssigkeit, ein Medikament oder dergleichen, zuzudosieren. In beiden Fällen bedarf es im Fluidsystem wiederverschließbarer Zugangsstellen, welche in der Praxis häufig als "Ports" bezeichnet werden.

US 4 230 128 A offenbart beispielsweise eine Befestigungsvorrichtung für einen Katheter, EP 1 234 596 A1 eine Vorrichtung zur Fluidströmungslenkung zwischen verschiedenen Kanälen und EP 1 555 041 A1 einen Injektionsadapter mit einem verdrehbaren Betätigungselement.

US 5 135 026 A offenbart eine Vorrichtung, um eine Fluidströmung zwischen verschiedenen Kanälen zu lenken. WO 2011/143049 A2 offenbart ein Ventil zum Steuern der Abgabe eines Fluids und aus der US 2010/319796 A1 ist ein "multiport valve" (eine Vorrichtung zum Verbinden von Fluidanschlüssen in unterschiedlichen Verbindungskombinationen) gemäß dem Oberbegriff des Anspruchs 1 bekannt.

Eine Aufgabe der vorliegenden Erfindung ist es, einen weiteren medizinischen Port vorzuschlagen. Zudem sollen ein Blutschlauch zur Verwendung bei einer extrakorporalen Blutbehandlung sowie eine medizinische Behandlungsvorrichtung angegeben werden.

Die erfindungsgemäße Aufgabe wird durch einen medizinischen Port mit den Merkmalen des Anspruchs 1 gelöst. Sie wird ferner gelöst durch einen Blutschlauch mit den Merkmalen des Anspruchs 12 sowie durch eine medizinische Behandlungsvorrichtung mit den Merkmalen des Anspruchs 13.

Der erfindungsgemäße Port weist einen Hauptkanal mit einem Lumen zum Leiten eines ersten Fluids durch den Port hindurch auf. Der Hauptkanal weist eine Nebenkanalmündung eines Nebenkanals zum Zugeben eines zweiten Fluids in den Hauptkanal auf. Der Port weist ferner wenigstens ein Gehäuseelement und wenigstens ein relativ zum Gehäuseelement aus einer ersten Position in eine zweite Position überführbar angeordnetes Betätigungselement auf. Weiter weist der erfindungsgemäße Port einen Dichtabschnitt auf, welcher angeordnet ist, um bei Überführen des Betätigungselements von einer Position in die andere zwischen einer ersten Stellung des Dichtabschnitts, in welcher der Dichtabschnitt die Nebenkanalmündung nicht verschließt oder bedeckt (offene Stellung oder Ventilstellung) und einer zweiten Stellung des Dichtabschnitts, in welcher der Dichtabschnitt die Nebenkanalmündung verschließt oder bedeckt (geschlossene Stellung oder Ventilstellung) verdrehbar zu sein.

Der erfindungsgemäße Blutschlauch ist geeignet und/oder vorgesehen und/oder ausgelegt zur Verwendung bei einer extrakorporalen Blutbehandlung. Dabei weist der Blutschlauch wenigstens einen Port nach einem der vorangegangenen Ansprüche auf.

Die erfindungsgemäße medizinische Behandlungsvorrichtung ist mit wenigstens einem erfindungsgemäßen Blutschlauch verbunden.

Erfindungsgemäße Ausführungsformen können eines oder mehrere der im Folgenden genannten Merkmale aufweisen. Auch die Gegenstände der Unteransprüche geben jeweils erfindungsgemäße Ausführungsformen an.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Das erste Fluid ist zumeist eine Flüssigkeit, beispielsweise Blut, Dialysierflüssigkeit oder Infusionslösung. Das zweite Fluid ist regelmäßig eine Behandlungsflüssigkeit, ein Medikament oder dergleichen. Sowohl das erste als auch das zweite Fluid können ferner Gase sein.

Der Hauptkanal ist in manchen erfindungsgemäßen Ausführungsformen einstückig in den erfindungsgemäßen Port integriert.

In einigen erfindungsgemäßen Ausführungsformen ist der Hauptkanal allein durch das Gehäuseelement ausgestaltet, liegt jedenfalls allein in diesem oder erstreckt sich nicht über dieses hinaus.

In manchen erfindungsgemäßen Ausführungsformen erstreckt sich der Hauptkanal nicht in an den Port angeschlossene Schlauchleitungen hinein.

Das Überführen des Betätigungselements von einer Position in die andere erfolgt in einigen erfindungsgemäßen Ausführungsformen durch dessen Drehen, beispielsweise um seine Längsachse.

Der Hauptkanal des Ports ist in bestimmten erfindungsgemäßen Ausführungsformen ein Rohr.

In manchen erfindungsgemäßen Ausführungsformen ist der Hauptkanal ausgestaltet als durchgehende und/oder einstückige Rohrstruktur.

Das Lumen des Hauptkanals weist in einigen erfindungsgemäßen Ausführungsformen keine Stufe bezogen auf seinen Längsschnitt auf.

In bestimmten erfindungsgemäßen Ausführungsformen weist der Hauptkanal keine Veränderung seines inneren Querschnitts oder seines Lumenquerschnitts auf. In anderen erfindungsgemäßen Ausführungsformen weist der Hauptkanal allenfalls kontinuierliche, nicht aber eine oder mehrere sprunghafte oder stufenhafte Querschnittsveränderungen auf.

In einigen erfindungsgemäßen Ausführungsformen wird das Betätigungselement unter seiner Drehung von seiner ersten Position in seine zweite Position übergeführt, wie dies auch bei den mittels der Figuren detaillierter erläuterten erfindungsgemäßen Ausführungsformen der Fall ist. Die Erfindung ist allerdings nicht hierauf beschränkt. So kann das Betätigungselement auch durch einen Schiebemechanismus oder durch einen Druckmechanismus betätigt werden. In jedem Fall aber verdreht sich bei Betätigung des Betätigungselements der Dichtabschnitt zwischen den verschiedenen, hierin genannten Stellungen.

In manchen Ausführungsformen des erfindungsgemäßen Ports ist der Dichtabschnitt angeordnet und ausgestaltet, um eine Durchgängigkeit des Hauptkanals für das erste Fluid weder in seiner ersten Stellung noch in seiner zweiten Stellung zu beeinträchtigen.

In bestimmten Ausführungsformen des erfindungsgemäßen Ports ist der Dichtabschnitt angeordnet und ausgestaltet, um eine Durchgängigkeit des Hauptkanals für das erste Fluid weder in seiner ersten Stellung noch in seiner zweiten Stellung auch nicht in Zwischenstellungen zwischen erster und zweiter Stellung und/oder beim Übergang zwischen erster und zweiter Stellung zu beeinträchtigen.

Unter einer Beeinträchtigung der Durchgängigkeit wird in gewissen erfindungsgemäßen Ausführungsformen jede Lumenveränderung oder Querschnittsveränderung des Hauptkanals und/oder jede Beeinflussung des im Hauptkanal strömenden Fluids, wie etwa dessen Strömungsgeschwindigkeit, seines Drosselungszustands, usw. verstanden.

Da ein Beeinträchtigung mit der Erzeugung oder Verstärkung von Turbulenzen einhergehen kann, kann eine ausbleibende Beeinträchtigung mit den für den Fachmann hiermit verbundenen Vorteilen verbunden sein.

Die Durchgängigkeit des Hauptkanals für das erste Fluid wird in einigen erfindungsgemäßen Ausführungsformen durch Überführen des Dichtabschnitts von der ersten Stellung in die zweite Stellung (im Folgenden auch als "Schließen des Ventils" bezeichnet) nicht verändert.

Der Ausdruck "beeinträchtigen" ist dabei in bestimmten erfindungsgemäßen Ausführungsformen z. B. als Vermindern, Behindern, Verringern, Verändern von Strömungswegen, etc. zu verstehen.

In manchen erfindungsgemäßen Ausführungsformen liegt in keiner Stellung des Dichtabschnitts ein Abschnitt des Dichtabschnitts im Lumen des Hauptkanals vor.

In einigen erfindungsgemäßen Ausführungsformen des Ports weist der Hauptkanal zusätzlich zur Nebenkanalmündung eine Septummündung auf, welche im Gebrauch des Ports mit einem mittels Kanüle durchstechbaren Septum verschlossen ist.

In manchen erfindungsgemäßen Ausführungsformen des Ports ist die Septummündung zusätzlich zur Nebenkanalmündung im Dichtabschnitt vorgesehen.

In einigen erfindungsgemäßen Ausführungsformen des Ports mündet die Septummündung oder deren Hauptdurchtrittsrichtung nicht parallel - sondern vorzugsweise im Wesentlichen oder vollständig rechtwinklig - zu einer Haupterstreckungsrichtung des Nebenkanalrohrs.

In bestimmten erfindungsgemäßen Ausführungsformen des Ports mündet die Nebenkanalmündung und/oder die Septummündung in einen gerade verlaufenden Abschnitt eines Querschnitts des Lumen des Hauptkanals oder eines Segments oder Abschnitts hiervon.

Unter einem gerade verlaufenden Abschnitt ist in einigen erfindungsgemäßen Ausführungsformen ein Abschnitt der Wandung des Hauptkanals mit einer Öffnung (für die Nebenkanalmündung oder die Septummündung) zu verstehen, welche sich ausschließlich eben erstreckt oder welche mit einer ebenen Fläche abzudichten ist. In gewissen erfindungsgemäßen Ausführungsformen ist die ebene Fläche, welche die Öffnung in deren gesamtem Umfang schneidet oder welche deren gesamten Rand enthält, senkrecht zu einer Drehachse des Dichtabschnitts angeordnet, um welche dieser von der ersten Stellung in die zweite Stellung gedreht wird.

In manchen erfindungsgemäßen Ausführungsformen des Ports mündet die Nebenkanalmündung und/oder die Septummündung jeweils ganz oder zumindest beispielsweise etwa zur Hälfte in einen gerade verlaufenden Abschnitt eines Querschnitts des Lumens (oder dessen Begrenzung) des Hauptkanals. Die andere Hälfte oder der andere Teil liegt hierbei in einem runden oder gerundeten oder gekrümmten Querschnittsabschnitt.

Die Nebenkanalmündung hat in bestimmten erfindungsgemäßen Ausführungsformen des Ports weder eine ausschließlich ebene noch eine ausschließlich gleichmäßig gekrümmte Öffnungsfläche.

Die Septummündung hat in bestimmten erfindungsgemäßen Ausführungsformen des Ports eine ausschließlich ebene Öffnungsfläche.

In manchen Ausführungsformen des erfindungsgemäßen Ports weist der Dichtabschnitt eine stirnseitige Abdichtfläche auf. Diese Abdichtfläche ist angeordnet, um bei einer Drehung des Dichtabschnitts entlang einer Drehkurve und/oder auf einer Drehkurve von einer ersten Stellung in eine zweite Stellung bewegt zu werden. Dabei verschließt oder bedeckt die Abdichtfläche in der zweiten Stellung die Nebenkanalmündung. In der ersten Stellung verschließt oder bedeckt die Abdichtfläche die Nebenkanalmündung nicht. Die Abdichtfläche erstreckt sich dabei parallel zu einer Hauptquerschnittsebene des Dichtabschnitts, also senkrecht zu dessen Dreh- oder Längsachse.

Erfindungsgemäß weist der Dichtabschnitt eine Dichtnase auf, welche in axialer Richtung des Dichtabschnitts - oder stirnseitig - über den Dichtabschnitt vorsteht. Dabei ist die Dichtnase angeordnet, um bei einer Drehung des Gehäuseelements, oder bei dessen Überführen von einer Position in die andere, auf einer Drehkurve von einer ersten Stellung in eine zweite Stellung auf der Drehkurve bewegt zu werden. Die Dichtnase verschließt oder bedeckt dabei in der zweiten Stellung die Nebenkanalmündung, in der ersten Stellung verschließt oder bedeckt die Dichtnase die Nebenkanalmündung nicht.

In bestimmten erfindungsgemäßen Ausführungsformen des Ports verschließt oder bedeckt die Dichtnase in der zweiten Stellung die Nebenkanalmündung z. B. mit einer zumindest teilweise ansteigend - z. B. bezogen auf eine Hauptquerschnittsebene des Dichtabschnitts - verlaufenden Abdichtfläche.

Das Gehäuseelement weist in manchen erfindungsgemäßen Ausführungsformen des Ports wenigstens einen (im Folgenden auch als "Schalttopf" bezeichneten) Abschnitt auf. Dieser Abschnitt weist eine Vertiefung zur Aufnahme der hierin bewegbaren Dichtnase auf. Er weist ferner die Nebenkanalmündung auf oder grenzt hieran an.

Dieser Abschnitt weist in bestimmten erfindungsgemäßen Ausführungsformen einen im Wesentlichen runden Querschnitt auf.

In manchen erfindungsgemäßen Ausführungsformen des Ports weist die Dichtnase eine sowohl zu einer Stirnseite der Dichtnase als auch zu einer lateralen Seitenfläche des Dichtabschnitts offene Nut auf.

Die offene Nut ist in einigen erfindungsgemäßen Ausführungsformen ein im Dichtabschnitt oder im Dichtelement angeordneter Nebenkanal oder ein Abschnitt hiervon.

In bestimmten erfindungsgemäßen Ausführungsformen des Ports liegt die Nut in der ersten Stellung des Dichtabschnitts derart an einer Öffnung eines Nebenkanalrohrs an, dass sie den Fluidweg des Nebenkanalrohrs über den Dichtabschnitt hinweg fortsetzt. Dabei steht die Nut in der zweiten Stellung des Dichtabschnitts nicht mit dem Nebenkanalrohr oder dessen Öffnung in Fluidverbindung.

In manchen erfindungsgemäßen Ausführungsformen des Ports weist der Dichtabschnitt zusätzlich zur stirnseitigen Abdichtfläche oder zusätzlich zur Dichtnase eine erhabene, in ihrem Umfang geschlossene Dichtstruktur auf, welche in der zweiten Stellung des Dichtabschnitts die Öffnung des Nebenkanalrohrs verschließt oder einen Austritt von Fluid aus der Öffnung verhindert.

"Erhaben" bedeutet in einigen erfindungsgemäßen Ausführungsformen, dass sich die Dichtstruktur über ein Niveau der Seitenfläche des Dichtabschnitts erhebt.

Die geschlossene Dichtstruktur liegt in manchen erfindungsgemäßen Ausführungsformen an einer Seitenfläche oder an einem Umfang der Dichtabschnitts.

Der Dichtabschnitt ist in bestimmten erfindungsgemäßen Ausführungsformen des Ports als ein separates Dichtelement ausgestaltet, in gewissen erfindungsgemäßen Ausführungsformen ist er einstückig ausgestaltet.

Das Dichtelement ist in manchen erfindungsgemäßen Ausführungsformen aus einem anderen Material gefertigt als das Gehäuseelement und/oder das Betätigungselement.

In einigen erfindungsgemäßen Ausführungsformen liegt das Dichtelement im Gebrauch zwischen dem Gehäuseelement und dem Betätigungselement.

In manchen erfindungsgemäßen Ausführungsformen des Ports weist der Dichtabschnitt wenigstens ein durchstechbares Septum auf.

In bestimmten erfindungsgemäßen Ausführungsformen des Ports sind sowohl die Septummündung als auch die Nebenkanalmündunggemeinsam in einer Querschnittshälfte des Hauptkanals angeordnet.

Der erfindungsgemäße Blutschlauch weist in manchen erfindungsgemäßen Ausführungsformen wenigstens eine arterielle Patientenleitung und wenigstens eine venöse Patientenleitung auf.

In einigen erfindungsgemäßen Ausführungsformen des Blutschlauchs ist der erfindungsgemäße Port in die arterielle Patientenleitung eingefügt.

In manchen erfindungsgemäßen Ausführungsformen ist die medizinische Behandlungsvorrichtung als Blutbehandlungsvorrichtung, insbesondere als Vorrichtung zur Apherese oder Dialyse, wiederum insbesondere zur Hämodialyse, Hämofiltration, Hämodiafiltration, Peritonealdialyse, Akutdialyse usw. ausgestaltet.

Einige oder alle erfindungsgemäßen Ausführungsformen können einen oder mehrere der oben oder im Folgenden genannten Vorteile aufweisen. Dabei wird zum besseren Verständnis auf die sich anschließende Figurenbeschreibung hingewiesen.

Der erfindungsgemäßen Port weist wenigstens einen Nebenkanal auf, der über eine verschließbare Öffnung mit dem Hauptkanal in Verbindung steht. Der Nebenkanal kann Fluidverbindungen zu weiteren Abschnitten eines Fluidsystems herstellen. Er kann beispielsweise zu einem Nebenkanalkonnektor führen, welcher vorteilhaft eine lösbare Fluidverbindung zu einem Fluidschlauch darstellt. Durch diesen Nebenkanal kann vorteilhaft ein weiteres Fluid in den Hauptkanal zugegeben oder Proben des ersten Fluids aus dem Hauptkanal entnommen werden.

Der Mechanismus, welcher die Verbindung des Nebenkanals zum Hauptkanal öffnet oder verschließt, verfügt zumindest über eine Stellung, in welcher der Hauptkanal vorteilhaft totraumfrei oder im Wesentlichen totraumfrei vom Nebenkanal abgetrennt ist.

Der erfindungsgemäße Port oder Kombiport kann wenigstens die zwei Ventilstellungen "Zu" und "Auf" aufweisen. Zusätzlich kann der Ventilmechanismus vorteilhaft zur stufenlosen und feingestuften Drosselung entnommener oder eingespeister Fluide dienen. Wahlweise kann eine der beiden Ventilstellungen oder eine dritte Ventilstellung vorteilhaft dazu verwendet werden, ein einstellbar vorgespanntes Rückschlagventil mit Freigabe ausschließlich für Zuströmungen einzurichten. Ferner ist die Ventilstellung vorteilhaft maschinell überwachbar.

In manchen erfindungsgemäßen Ausführungsformen ist der erfindungsgemäße Port ausgestaltet, um in oder an einer Aufnahme aufgenommen zu werden. Die Aufnahme kann beispielsweise an der Front oder einem anderen Abschnitt, insbesondere des Gehäuses, der Behandlungsvorrichtung vorgesehen sein. Die Aufnahme dient vorzugsweise dazu, den Port lösbar zu fixieren. Dieser kann beispielsweise verrastet, verschnappt, geklemmt, gehaltert, und dergleichen werden. Der Anwender kann so die Ventilstellung des Ports gemeinsam oder in Zusammenschau mit anderen Einrichtungen oder Anzeigen, sozusagen auf einen Blick und im Kontext mit diesen, sehen.

Zudem ist es möglich und in manchen erfindungsgemäßen Ausführungsformen vorgesehen, die Existenz des Ports an der Behandlungsvorrichtung (beispielsweise in oder an der Aufnahme) und/oder dessen Ventilstellung automatisch durch die medizinische Behandlungsvorrichtung ablesen zu lassen. Eine entsprechende Einrichtung, welche beispielsweise mittels optischem Sensor, Farbsensor, oder dergleichen ausgestattet ist, kann an der Behandlungsvorrichtung vorgesehen sein. Die hierbei gewonnene Information kann in der Steuerung oder Regelung der Behandlungsvorrichtung weiterverwendet werden.

Der erfindungsgemäße Port besteht in einigen erfindungsgemäßen Ausführungsformen vorteilhaft aus nur drei Komponenten. Dadurch wird eine kompakte Verpackungsanordnung ermöglicht.

Zudem erlaubt der erfindungsgemäße Port in einigen erfindungsgemäßen Ausführungsformen auf wenigstens einen bei herkömmlichen Portlösungen eingesetzten Schlauch oder Schlauchabschnitt zu verzichten. Hierdurch kann es nicht zum Ausknicken dieses Schlauchs kommen.

Durch die geringe Baulänge benötigt der Port vorteilhaft wenig Platz während seines Gebrauchs.

Die bisher aus dem Stand der Technik bekannten Zugangsstellen weisen Strömungstoträume auf zwischen dem Lumen des Hauptkanals und dem dichtenden Septum, oder zwischen dem Lumen des Hauptkanals und dem Absperrventil des Nebenkanals. Diese Toträume verursachen Luftnester bei der Befüllung des Fluidsystems über den Hauptkanal. Das erste durch den Hauptkanal strömende Fluid, zumeist Blut, dringt ferner in diese Toträume vor. Blut kann dort aufgrund mangelnder Strömung und aufgrund des Kontakts des Bluts mit Luft leicht gerinnen. Dies wird durch den erfindungsgemäßen Port in manchen Ausführungsformen vorteilhaft vermieden.

Da ein solches Eindringen in (erfindungsgemäß nicht vorhandene oder zumindest minimierte) Toträume erfindungsgemäß vermieden wird, wird bei erstmaliger - aber auch noch bei späterer - Zugabe eines zweiten Fluids über die Nebenkanalmündung oder über das Septum in den Hauptkanal derart geschädigtes, da geronnenes Blut nicht in den Hauptkanal zurückgeführt, worin ein weiterer Vorteil besteht.

Ebenso wird dadurch bei erstmaliger Verwendung der Zugangsstelle vorteilhaft verhindert, dass ein Luftbolus über die Nebenkanalmündung in den Hauptkanal infundiert, wie dies von Bauformen mit herkömmlichem Schlauchstück und Schlauchklemme bekannt ist.

In bilanzierenden Anordnungen müssen Volumenströme möglichst genau festlegbar sein. Durch die Kompressibilität der Luftsäule in Strömungstoträumen können Verfälschungen der Volumenströme und schwankende Ergebnisse bei Druckmessungen verursacht werden. Dies ist erfindungsgemäß nicht der Fall, da Toträume erfindungsgemäß vermeidbar sind.

Der erfindungsgemäße Port kommt ferner ohne eine Schlauchklemme zum Absperren des Nebenkanals aus. Dies ist aus verschiedenen Gründen vorteilhaft. Zum einen ist die Schlauchklemme erfindungsgemäß nicht erforderlich, was Kosten und Mühen einsparen kann. Zudem wird bei Schlauchklemmen häufig beobachtet, dass sie, wenn sie für lange Zeit geschlossen sind, was bei Auslieferung des Fluidsystems mit geschlossenen Schlauchklemmen der Fall ist, ermüden. Dies gilt auch für das Material des abgeklemmten Schlauchabschnitts. Insbesondere führt eine lange geschlossene Schlauchklemme immer wieder dazu, dass sich der Schlauch bei Öffnung der Schlauchklemme entweder gar nicht oder nur mehr unvollständig öffnet. Es verbleibt in aller Regel an der Klemmstelle eine Eindellung, welche die Knickneigung an dieser Stelle signifikant erhöht. Diese Nachteile werden durch den erfindungsgemäßen Port ohne Schlauchklemme vorteilhaft vermieden.

In geschlossener Stellung eignen sich Fluidsysteme mit Schläuchen und Schlauchklemmen aus kostengünstigen thermoplastischen Materialien generell nicht für eine Sterilisation mit Dampf, da beide Bestandteile durch die hierbei übliche Temperatur von 121°C bleibend geschädigt werden. Die daher anfänglich notwendigerweise offene (Initial-)Stellung der Schlauchklemmen erfordert entweder eine zweite Absperrung, etwa eine dichte Luer-Schutzkappe. Oder sie erfordert, dass der Anwender beim Aufrüsten der Behandlungsvorrichtung keineswegs vergessen darf, die Schlauchklemmen vor Gebrauch des Ports zu schließen. Dies birgt das Risiko, dass dies vergessen wird.

Bei Dampfsterilisation sind grundsätzlich Luer-Schutzkappen ohne Dichtfunktion erforderlich. Diese enthalten eine in der Regel von außen nicht oder nur schwer wahrnehmbare Dampfdurchlassstelle. Da die überwiegende Anzahl der marktüblichen Fluidsysteme nicht dampfsterilisiert sind, ist dem Anwender nur schwer zu vermitteln, dass er sich auf die Dichtwirkung der Luer-Schutzkappen nicht verlassen kann sondern alle Klemmen schließen muss, was er bei anderen Systemen erst tun muss, bevor er die Schutzkappen entfernt.

Diese Unsicherheiten und Risiken entfallen bei Verwenden des erfindungsgemäßen Ports: Der erfindungsgemäße Port ist vorteilhaft in allen Ventilstellungen sterilisier- und lagerbar.

Da der erfindungsgemäße Port in bestimmten erfindungsgemäßen Ausführungsformen keine Toträume aufweist, steht sein Einsatz in einem Fluidsystem nicht einer reversen arteriellen Blutrückgabe nach Behandlungsende entgegen. Die für eine solche Blutrückgabe erforderliche totraumfreie Anordnung, da bei diesen Verfahren weder eine Luftabscheidekammer noch ein Gerinnselfänger oder ein Luftdetektor Luft und geschädigtes Blut daran hindern, aus dem Totraumbereich in den menschlichen Körper zu gelangen, wird vom erfindungsgemäßen Port vorteilhaft gewährleistet.

In einer bevorzugten Ausführungsform ist das Gehäuseelement mit dem Hauptkanal und daher vorteilhaft besonders kostengünstig aus Thermoplasten spritzgießbar.

Die vorliegende Erfindung stellt vorteilhaft einen wiederholbar verschließ- und öffenbaren Port bereit. Dieser erlaubt auch während der Behandlung des Patienten Konnektionen und Dekonnektionen ohne Flüssigkeitsverlust und ohne ungewollten Flüssigkeits- oder Lufteintritt in die Hauptströmung.

Ein weiterer Vorteil dieser Herstellung ergibt sich daraus, dass weder geklebt noch geschweißt werden muss. Ferner kann die Herstellung ohne weiteres ausschließlich automatisch oder maschinell erfolgen.

Bei einigen erfindungsgemäßen Ausführungsformen des Ports weist dieser zusätzlich ein selbstverschließendes Stechseptum auf. Hierüber kann totraumfrei mittels Kanüle ein Fluid aus dem Hauptkanal entnommen oder diesem zugeführt werden.

Die Dichtungen des erfindungsgemäßen Ports sind in einigen Ausführungsformen vorteilhaft bidirektional selbstverstärkend.

Die Verwendung von Polypropylen (PP), wie in manchen Ausführungsformen vorgesehen, erlaubt eine preisgünstige, umweltneutrale und bioneutrale Herstellung des Ports. PP ist zudem vorteilhaft mit allen bekannten Verfahren sterilisierbar, d. h. gas-, dampf- und strahlensterilisierbar.

Die Betätigung des Ports und insbesondere des Betätigungselements ist vorteilhaft für Links- wie Rechtshänder gleichermaßen einfach möglich.

Die vorliegende Erfindung erlaubt vorteilhaft eine schon von weitem sichtbare Ventilstellung.

Eine sensorische Überwachung der aktuellen Ventilstellung durch die Behandlungsvorrichtung ist bei der vorliegenden Erfindung vorteilhaft optional realisierbar, ebenso die mögliche aktive Blockade einer Ventilstellung durch die Vorrichtung oder die maschinelle Betätigung der Ventilstellung. Auf diese Weise kann ein neues Niveau bei der Behandlungssicherheit und bei der Benutzerführung erreicht werden.

Durch die Integration von Nebenkanalmündung und Septummündung in ein einziges oder in wenige Bauteile wird vorteilhaft die Funktionssicherheit erhöht und der Herstellaufwand vermindert.

Ferner sind die zueinander drehenden Teile, also Gehäuseelement, Betätigungselement und Dichtelement, vorteilhaft permanent gegen Fluidaustritt und Fluideintritt (Gase und Flüssigkeiten) zwischen dem ersten Fluid und der Umgebung, und umgekehrt, abgedichtet. Dies wird in bestimmten erfindungsgemäßen Ausführungsformen mittels einer permanent vorgespannten radialen, halbaxialen oder axialen Dichtanordnung erzielt. Der Dichtabschnitt, oder das Dichtelement, übernimmt somit auch diese Stopfbuchsdichtfunktion. Weitere Dichtungen, also neben den jeweils einstückigen Drehpartnern, sind vorteilhaft nicht erforderlich.

Die vorliegende Erfindung schlägt vorteilhaft eine Rücktrocknungsdrainage für die Septummündung vor.

Eine Rücktrocknungsdrainage weist in manchen erfindungsgemäßen Ausführungsformen eine oder mehrere kapillare Struktur(en) auf - oder besteht hieraus - zum Leiten oder Führen von Flüssigkeit von einem Funktionselement (z. B. dem Septum) weg oder zu diesem hin.

Die vorliegende Erfindung umfasst in einigen Ausführungsformen vorteilhaft einen doppelten Schutz davor, dass das Septum versehentlich berührt wird.

Die von der vorliegenden Erfindung umfasste Kanülenführung weist vorteilhaft einen Schutz gegen fehlerhaftes Einstechen in das Septum auf.

Die vorliegende Erfindung bietet somit vorteilhaft Schutz vor Verschleppung von Partikeln über das Septum hinweg in das erste Fluid.

Die vorliegende Erfindung weist vorteilhaft eine einfache, zuverlässige Aufschnapp-Halterung für Vorrichtungen auf.

Eine von der Ventilstellung schaltstellungsabhängige Sicht auf Stellungspiktogramme ist durch die vorliegende Erfindung vorteilhaft umfasst.

Die vorliegende Erfindung erlaubt aufgrund ihrer Ausgestaltung vorteilhaft eine farbliche Kodierung ihres Betätigungselements, da dieses nicht in Kontakt mit dem ersten Fluid steht.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1a: zeigt einen Port einer ersten erfindungsgemäßen Ausführungsform in leicht perspektivischer Ansicht in der zweiten Stellung oder der geschlossenen Ventilstellung;
- Fig. 1b: zeigt den Port der Fig. 1a in der ersten Stellung oder der offenen Ventilstellung;
- Fig. 1c: zeigt einen Port einer zweiten erfindungsgemäßen Ausführungsform von vorne in geschlossener Ventilstellung;
- Fig. 1d: zeigt den Port der Fig. 1c in offener Ventilstellung;
- Fig. 1e: zeigt einen erfindungsgemäßen Port einer weiteren Ausführungsform von vorne;
- Fig. 1f: zeigt den erfindungsgemäßen Port der Fig. 1a und 1b in Explosionsdarstellung;
- Fig. 2a: zeigt den erfindungsgemäßen Port der ersten erfindungsgemäßen Ausführungsform in einem Längsschnitt in geschlossener Ventilstellung;
- Fig. 2b: zeigt den Port der Fig. 2a in offener Ventilstellung;
- Fig. 3: zeigt den Port der Fig. 2a leicht perspektivisch bei durchstochenem Septum;
- Fig. 4: zeigt die wesentlichen Fluidwege innerhalb des Ports der Fig. 1a in einem Längsschnitt;
- Fig. 5a: zeigt einen Peripheralteilschnitt durch den erfindungsgemäßen Port in der geschlossenen Ventilstellung;
- Fig. 5b: zeigt einen vollständigen Peripheralschnitt durch den erfindungsgemäßen Port der Fig. 5a in der offenen Ventilstellung, leicht gedreht gegenüber der Darstellung der Fig. 5a;
- Fig. 6a: zeigt den Port der Fig. 1a in einem Frontalschnitt des Betätigungselements;
- Fig. 6b: zeigt den Port der Fig. 6a in offener Ventilstellung;
- Fig. 7a: zeigt den erfindungsgemäßen Port unmittelbar vor einem Verrasten des Betätigungselements mit dem Gehäuseelement in einem Teilschnitt;
- Fig. 7b: zeigt den Port der Fig. 7a in einem verrasteten Zustand;
- Fig. 8: zeigt das Gehäuseelement des Ports der ersten Ausführungsform in perspektivischer Ansicht;
- Fig. 9: zeigt das Gehäuseelement des Ports der ersten Ausführungsform in leichter Perspektive im Längsschnitt durch das Nebenkanalrohr des Nebenkanals;
- Fig. 10: zeigt das Gehäuseelement einer weiteren Ausführungsform in einem Längsschnitt durch den Nebenkanal, wiederum in perspektivischer Ansicht;
- Fig. 10a: zeigt eine Ansicht von schräg oben in einen Ausschnitt des Schalttopfs mit einer Vertiefung für eine Dichtnase;
- Fig. 11a: zeigt die Unterseite des Betätigungselements eines erfindungsgemäßen Ports in einem Querschnitt, in perspektivischer Ansicht;
- Fig. 11b: zeigt das Betätigungselement einer wiederum weiteren Ausführungsform des Ports in einem Querschnitt, wiederum in perspektivischer Sicht mit Blick auf die Oberseite;
- Fig. 12a: zeigt das Dichtelement eines erfindungsgemäßen Ports von der Fluidseite aus betrachtet in leicht perspektivischer Ansicht;
- Fig. 12b: zeigt das Dichtelement der Fig. 12a von der Seite des Betätigungselements aus betrachtet;
- Fig. 13: zeigt eine weitere Ausgestaltung des Dichtelements in perspektivischer Ansicht;
- Fig. 14a: zeigt eine erfindungsgemäße Ausführungsform des Ports mit doppelter Abdichtung in einem Frontalschnitt bei geschlossener Ventilstellung;
- Fig. 14b: zeigt den Port der Fig. 14a in offener Ventilstellung;
- Fig. 15a: zeigt einen erfindungsgemäßen Port einer weiteren Ausführungsform in der geschlossenen Ventilstellung; und
- Fig. 15b: zeigt den erfindungsgemäßen Port der Fig. 15a in der offenen Ventilstellung.

Der im Folgenden exemplarisch beschriebene erfindungsgemäße Port weist einen Hauptkanal für ein Fluid auf und ist vorgesehen, um mittels diesem in eine Schlauchleitung integriert zu werden. Der Port weist ferner eine Septum-Zugangsstelle und eine Nebenzugangsstelle auf. Die Nebenzugangsstelle ist schaltbar ausgestaltet, sie ist beispielsweise eine Luer-Port-Zugangsstelle. Der Zugang über die Nebenzugangsstelle kann geöffnet (offene oder erste Stellung des Dichtabschnitts, auch als offene Ventilstellung bezeichnet) oder verschlossen (geschlossene oder zweite Stellung des Dichtabschnitts, auch als geschlossene Ventilstellung bezeichnet) werden. Der Dichtabschnitt ist mittels eines Betätigungselements des Ports zwischen wenigstens diesen beiden Stellungen schaltbar.

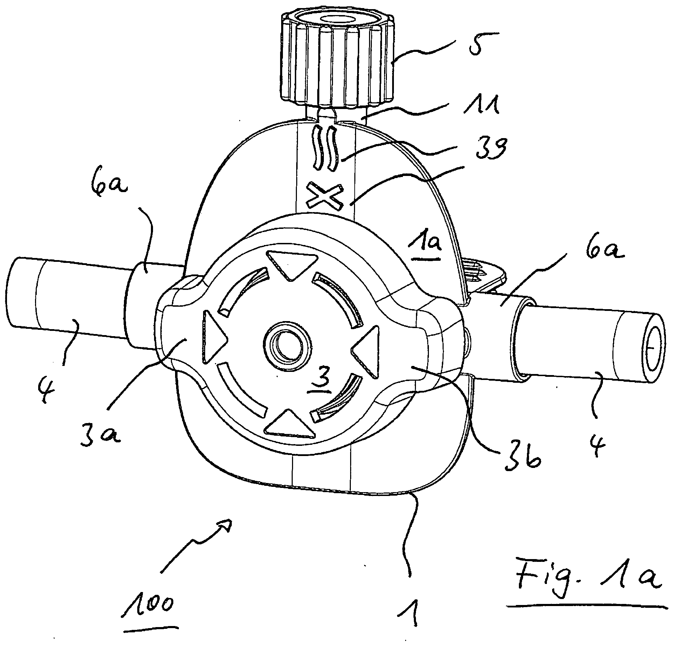

Die Fig. 1a bis 1f zeigen zwei voneinander verschiedene, jeweils erfindungsgemäße Ports oder Kombiports, hier als Port 100 bezeichnet, z. T. in unterschiedlichen Ventilstellungen, z. T. in unterschiedlichen Ansichten.

Fig. 1a zeigt in perspektivischer Ansicht den erfindungsgemäßen Port 100 einer ersten Ausführungsform mit einem Gehäuseelement 1 mit einer Stechschutzplatte 1a, einem Betätigungselement 3, einer auf einem Nebenkanalrohr 11 sitzenden Schutzkappe 5, und einem Hauptkanal 6. Der Hauptkanal 6 ist mittels zweier Schlauchmuffen 6a mit Anschlussschläuchen 4 verbunden.

Das Gehäuseelement 1 besteht in bestimmten Ausführungsformen aus einem spritzgegossenen Thermoplast oder weist einen solchen auf. Das Gehäuseelement 1 kann einstückig sein.

Der in den Fig. 1a bis 1e nicht dargestellte Dichtabschnitt, auch als Ventil bezeichnet, des erfindungsgemäßen Ports 100 befindet sich aufgrund der in Fig. 1a gezeigten zweiten Position des Betätigungselements 3 in einer geschlossenen, zweiten Stellung (geschlossene Stellung oder geschlossene Ventilstellung). Die geschlossene Stellung wird mittels Piktogramme 39 angezeigt, welche zur optischen Anzeige der Ventilstellung vorgesehen sind.

Die Schutzkappe 5 verschließt eine Öffnung eines in Fig. 1a nicht mittels Bezugszeichen bezeichneten Nebenkanalkonnektors des Nebenkanalrohrs 11.

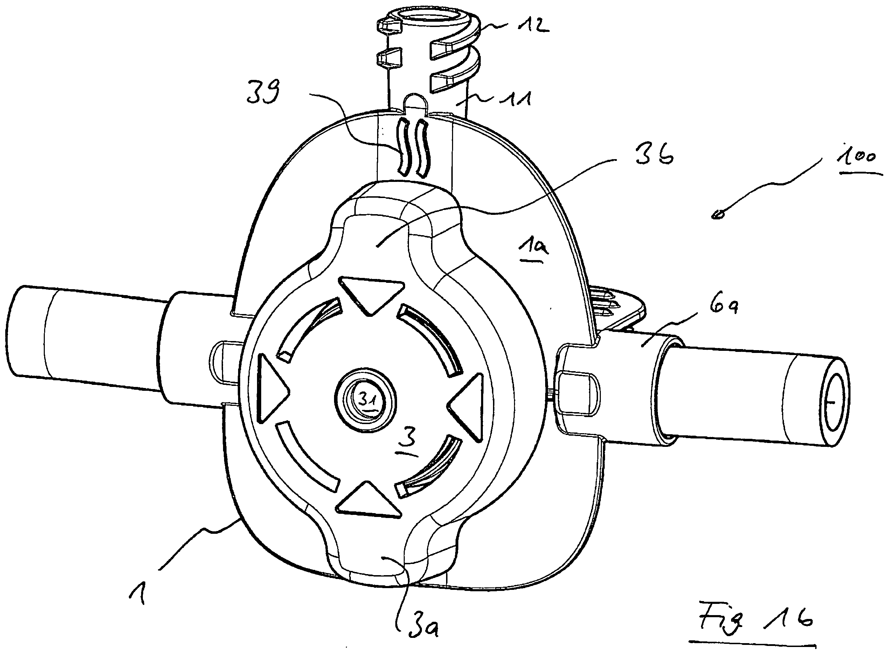

In Fig. 1b steht der nicht dargestellte Dichtabschnitt bzw. das Ventil aufgrund der vom Betätigungselements 3 eingenommenen ersten Position in der offenen oder durchgängigen ersten Stellung (offene Stellung oder Ventilstellung). Die offene Stellung ist wiederum taktil und optisch anhand der vom Betätigungselement 3 nicht bedeckten Abschnitte der Piktogramme 39 erkennbar. Die Piktogramme 39 zeigen mittels Wellenform an, dass in jeder Stellung des Betätigungselements 3 der Hauptkanal 6 frei durchströmt wird. In dieser Ausführung verdeckt das Betätigungselement 3 in seiner ersten Position ein Stop-Symbol ("X") und lässt nur mehr ein Flow-Symbol sichtbar. Dieses zeigt an, dass der Nebenkanal aufgrund der offenen Stellung des Dichtabschnitts, welche dieser immer dann einnimmt, wenn das Betätigungselement 3 in der ersten Position steht, ebenfalls durchströmt wird, oder dass eine Fluidverbindung zwischen Nebenkanal und Hauptkanal 6 besteht.

In Fig. 1b ist der erfindungsgemäße Port 100 bei abgenommener Schutzkappe 5 gezeigt. Der in Fig. 1a von der Schutzkappe 5 bedeckte Nebenkanalkonnektor 12, welcher hier exemplarisch als ein Luer-Port ausgestaltet ist, liegt nun frei. In der offenen Ventilstellung kann beispielsweise mittels Schlauch ein zweites Fluid über das Nebenkanalrohr 11 in den Port 100 eingebracht werden. Der Nebenkanalkonnektor 12 kann zum Verbinden des Schlauchs mit dem Port 100 dienen.

Neben dem Vorsehen von Piktogrammen 39, und unabhängig von ihrem Vorsehen, weist aber auch die äußere Formgebung des Betätigungselements 3 und seine jeweilige Drehstellung in einigen erfindungsgemäßen Ausführungsformen auf die jeweils vorliegende Ventilstellung hin. So erstreckt sich in Fig. 1b, anders als in Fig. 1a, eine Verbindungslinie zwischen Knebeln 3a und 3b des Betätigungselements 3 in geöffneter Ventilstellung in jener Richtung, in welche sich auch das Nebenkanalrohr 11 erstreckt. Damit deutet bereits die vom Betätigungselement 3 der Fig. 1b eingenommene Position intuitiv die offene Ventilstellung an; jedenfalls kann an der Stellung des Betätigungselements 3 die Ventilstellung abgelesen werden. Aufgrund der Formgebung des Betätigungselements 3 kann die Ventilstellung zudem bereits aus beachtlichem Abstand zu erkennen sein.

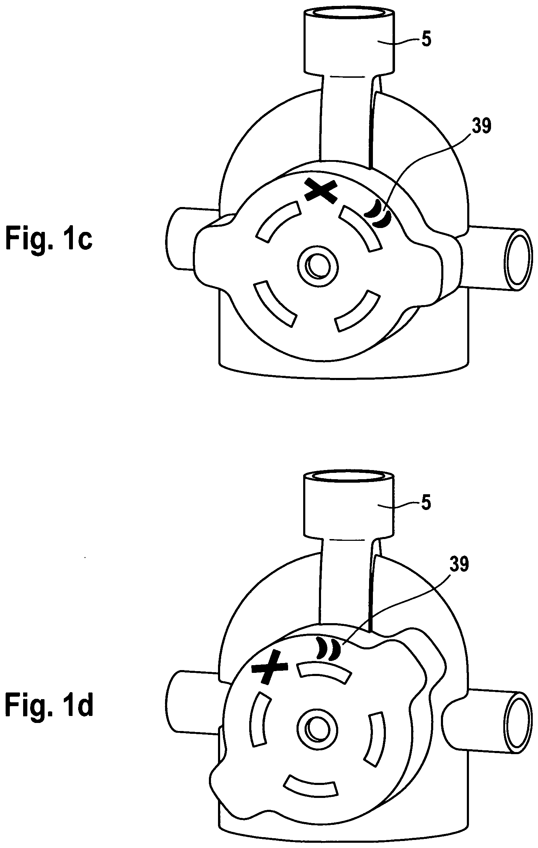

Die Fig. 1c und 1d zeigen eine zweite erfindungsgemäße Ausgestaltung des Ports 100 mit einer gegenüber der ersten Ausführungsform abweichenden Anordnung der Piktogramme 39. Diese sind in Fig. 1c und 1d an oder auf dem Betätigungselement 3 vorgesehen. Ferner weist der Aufdruck von vier Pfeilen, welche ebenfalls an oder auf dem Betätigungselement 3 vorgesehen sind, auf eine im Zentrum des Betätigungselements 3 gelegene Septum-Einstechmündung und einen Einstichkanal 31 hin. Sowohl das Gehäuseelement 1 als auch das Betätigungselement 3 können somit bereits durch die o. g. Piktogramme 39 die Bedienung des Ports 100 erleichtern und die Sicherheit bei seiner Bedienung erhöhen.

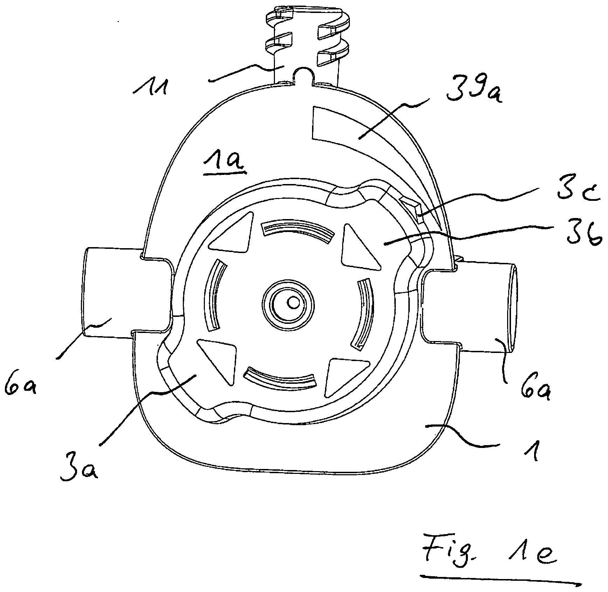

Das Ventil des erfindungsgemäßen Ports 100 kann in bestimmten Ausführungsformen zusätzlich in einer gedrosselten Durchflussstellung stehen, also weder vollständig geschlossen noch vollständig geöffnet sein, und daher zumindest einen geringen Fluss aus dem Hauptkanal 6 in das Nebenkanalrohr 11 hinein erlauben (oder umgekehrt). Das Ausmaß der Drosselung kann ebenfalls mittels einer entsprechenden Ausgestaltung der Piktogramme 39, etwa in Gestalt eines Pfeils mit zunehmender Stärke, angezeigt werden. Fig. 1e zeigt ein Beispiel einer solchen Kennzeichnung. So zeigt in Fig. 1e ein zusätzlicher Pfeil 3c auf dem Betätigungselement 3 auf einen entsprechenden Abschnitt eines Crescendo-Symbols 39a hin und gibt so den aktuellen Drosselungsgrad bei einer Ausgestaltung des Ports 100 mit kontinuierlichen Zwischenstellungen an.

Ferner geben weiter unten beschriebene deutliche Rastmomente zwischen den Endstellungen des Betätigungselements 3 und steife Drehanschläge dem Benutzer eine verlässliche haptische Rückmeldung über den Drehvorgang sowie über die hierdurch bewirkte Ventilstellung.

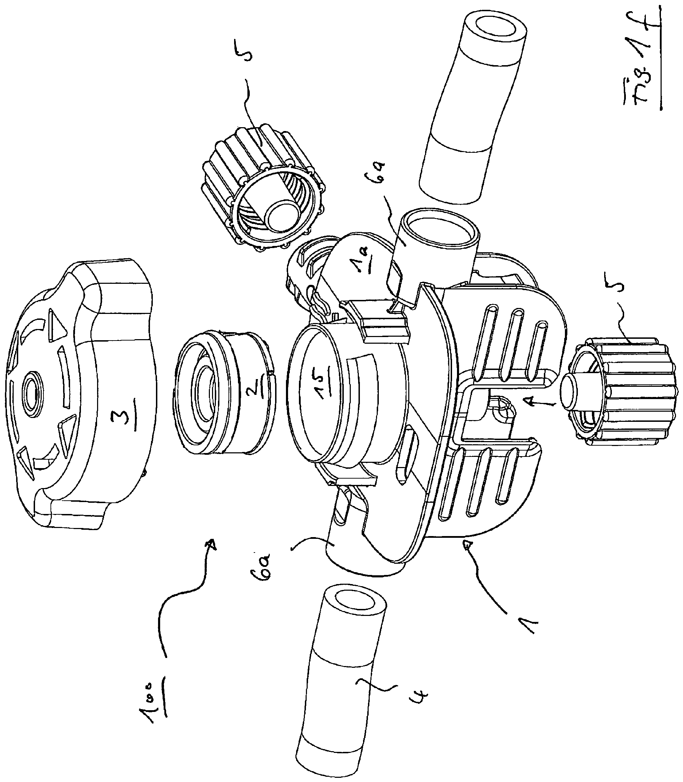

Fig. 1f zeigt eine Explosionsdarstellung des in den Fig. 1a und 1b gezeigten Ports 100. Zusätzlich zu den in den vorangegangenen Figuren gezeigten Elementen sind ein Dichtelement 2 - als Beispiel einer Ausführung eines separat vorliegenden Dichtabschnitts - und eine hier als Schalttopf 15 bezeichnete Struktur des Gehäuseelements 1 zu erkennen.

Der Schalttopf 15 weist eine Längsachse, welche zugleich dessen Symmetrieachse und/oder Drehachse sein kann, auf. Die Achse erstreckt sich, insbesondere im Wesentlichen oder vollständig, senkrecht bis diagonal zur Längsachse des weiter unten erläuterten Hauptkanals 6, welche wiederum dessen Symmetrieachse sein kann. Der Schalttopf 15 ist, vorzugsweise ganz oder im Wesentlichen, zylindrisch ausgestaltet oder hat zylindrische Abschnitte. Er kann Führungs- und/oder Abdichtpartner für das Dichtelement 2 und/oder für das Betätigungselement 3 sein. Aufgrund seiner Geometrie erlaubt der Schalttopf 15 Rotationsbewegungen des mit ihm verbundenen Dichtelements 2 und/oder des mit ihm verbundenen Betätigungselements 3.

Das Dichtelement 2 besteht in bestimmten Ausführungsformen aus einem gespritzten oder gepressten Elastomer. Elastomere zeichnen sich vorteilhaft durch besonders geringes Kriechverhalten unter Last aus. Dadurch kann eine gleichbleibende Dichtwirkung über die Einsatzzeit des Ports 100 auch nach längerer Lagerung des Ports 100 gewährleistet werden.

Bei einigen erfindungsgemäßen Ausführungsformen, bei denen ausschließlich ein Nebenkanal geschaltet oder gedrosselt wird, kommt vorteilhafterweise ein spritzgießbarer Silikongummiwerkstoff zum Einsatz, da dieser besonders präzise herstellbar und kriecharm ist.

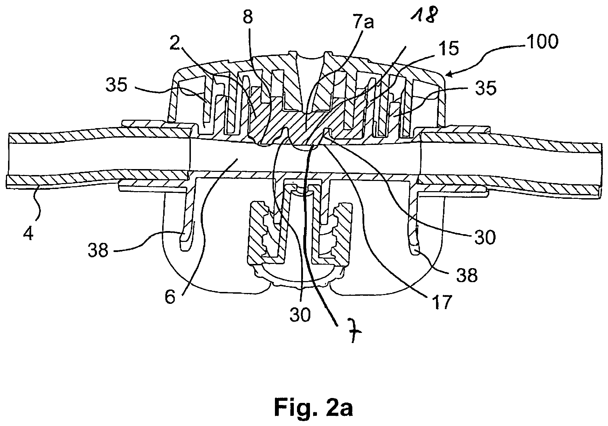

Bei manchen erfindungsgemäßen Ausführungsformen, bei welchen der Dichtabschnitt oder das Dichtelement 2 ferner einen Zugang zum Hauptkanal 6 mittels Septum 7a (siehe Fig. 2a) gewähren soll, kommen bevorzugt Elastomermischungen auf Basis von Isoprenkautschuk zum Einsatz. Diese Elastomere eignen sich besonders gut zum erneuten Abdichten nach dem Herausziehen einer Kanüle aus dem Septum 7a.

Bei Ausführungsformen mit geringen Anforderungen an Kriechbeständigkeit, Dichtdruck oder Wiederverschluss, insbesondere für Anwendungen, in denen keine Sterilisationsbehandlungen mit Wasserdampf erforderlich sind, können vorzugsweise auch thermoplastische Elastomere verwendet werden. Diese zeichnen sich durch einen geringeren Materialpreis und durch die Herstellbarkeit im preisgünstigen thermoplastischen Spritzgießverfahren aus.

Bei allen genannten Werkstoffen können am fertigen Dichtelement 2 vorteilhaft Nachbehandlungen in Form von Nachtempern, Waschen oder durch Beschichten mit Silikonölen erfolgen. Auf diese Weise können die Kriechbeständigkeit und die Partikelfreiheit erhöht und die Haftreibung und die Dichtwirkung gegenüber anderen Elementen verbessert werden.

Die Betätigungselemente 3 bestehen in manchen erfindungsgemäßen Ausführungsformen ebenfalls aus einem spritzgegossenen Thermoplast oder weisen einen solchen auf.

In einigen erfindungsgemäßen Ausführungsformen, wie z. B. der in Fig. 1a gezeigten, sind eine Mehrzahl von Funktionen im Betätigungselement 3 integriert. Zu diesen Ausgestaltungen zählen die gut greifbaren, ergonomisch drehbaren Knebel, aufgeprägte Piktogramme, Rastnasen zur permanenten Verrastung mit dem Gehäuseelement, Drehanschläge, Drehrastungen, Nasen zur Sensorüberwachung und weitere.

Das Betätigungselement 3 der Fig. 1a bis 1f ist vorteilhaft in einfachen Auf-Zu-Spritzgießwerkzeugen preisgünstig und reproduzierbar herstellbar.

Da kein Teil der Oberfläche des Betätigungselements 3 mit den zu behandelnden Flüssigkeiten wie Blut in Kontakt steht, kann das Betätigungselement 3 vorteilhaft eingefärbt werden. Besondere, verträgliche Pigmente sind vorteilhaft nicht erforderlich.

In Fig. 1f ist ferner eine Aufnahme zum vorübergehenden Aufnehmen oder Befestigen der Schutzkappe 5 am Port 100 gezeigt. An ihr lässt sich die Schutzkappe 5 aufbewahren, solange diese nicht benötigt wird. Eine solche Aufnahme für die Schutzkappe 5 kann selbstverständlich auch geeignet sein, Schutzkappen anderer medizinischer Vorrichtungen aufzunehmen. Die aufgenommene Schutzkappe muss nicht jene sein, mit welcher der Nebenrohrkonnektor 12 bedeckt wird. Sie muss nicht einmal Teil des erfindungsgemäßen Ports 100 sein.

In der Ausführungsform der Fig. 1a bis 1f sind nur die drei Komponenten Gehäuseelement 1, Dichtelement 2 und Betätigungselement 3 wesentlich. Die zusätzlich dargestellten Elemente wie die Schutzkappe 5 sind rein optional.

In anderen als der in den Fig. 1a bis 1f gezeigten Ausführungsform ist ferner auch das Dichtelement 2 nicht erforderlich. So kann beispielsweise für den Fall, dass das Dichtelement 2 aus, oder mit, thermoplastischen Elastomeren herstellt ist, das Dichtelement 2 - beispielsweise durch Aufschweißen (nach Pigmentierung eines Fügepartners beispielsweise auch über das Laser-Absorptions-Schweißen) oder durch Zweikomponenten-Spritzguss oder auf andere Weise oder einstückig - integral mit wahlweise dem Gehäuseelement 1 oder dem Betätigungselement 3 hergestellt sein. Diese stoffschlüssige Verbindung kann Vorteile bieten, beispielsweise kann die Genauigkeit beim Einstellen der einen oder der anderen Stellung des Dichtabschnitts (hier auch als Schaltwinkel bezeichnet) erhöht werden, Material eingespart werden, da die Eigensteifigkeit des Dichtelements niedriger sein kann, oder Herstellzeit eingespart werden, da Sortier- und Handlingvorgänge entfallen.

Der in den Fig. 1a bis 1f nur angedeutete Hauptkanal 6 ist als im Umfang geschlossenes Rohr ausgeführt. Das Rohr weist koaxiale, an den Enden angeordnete weibliche Schlauchmuffen 6a auf. Der Hauptkanal 6 ist mittels seiner Schlauchmuffen 6a beidseitig über hierin eingeklebte oder hieran verschweißte Abschlussschläuche 4 mit einem Fluidsystem verbunden, beispielsweise mit einem erfindungsgemäßen Blutschlauch. Das Gehäuseelement 1 bildet den größten Teil oder den gesamten Teil der fluidführenden Strukturen des Hauptkanals 6, des in den Fig. 1a bis 1f nicht zu erkennenden Nebenkanals und des Zugangs zum ebenfalls nicht dargestellten Septum.

Ist das Gehäuseelement 1 beispielsweise in einstückige Kassetten-Einmalteil-Fluidsysteme integriert, so kann es vorteilhaft möglich sein, den Hauptkanal 6 und/oder den Nebenkanal nicht als geschlossenes Rohr, sondern in geteilter bzw. halboffener Form auszubilden. Auf diese Weise sind andere Anbindungen der Fluidwege des Ports 100 an die weiteren Fluidstrukturen des Fluidsystems möglich. Beispielsweise können Hauptkanäle mit nicht geraden Strömungsachsen vorgesehen sein. Der Verschluss der halboffenen Kanalstrukturen kann beispielsweise durch weitere spritzgegossene Elemente oder durch aufgepresste, aufgeklebte oder aufgeschweißte Folien erfolgen.

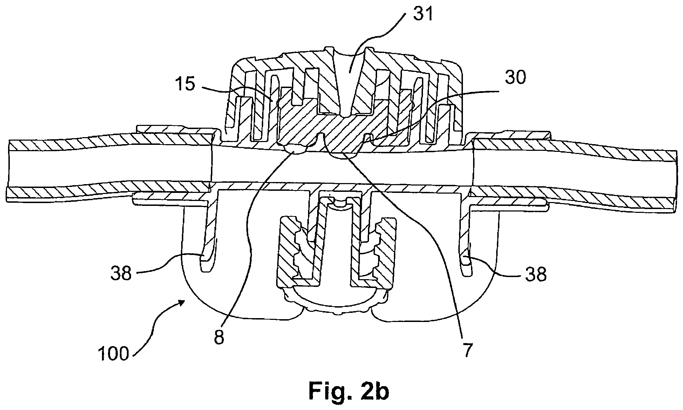

Der Hauptkanal 6 ist vorzugsweise als möglichst kurzes Rohr ausgebildet. Wie aus Fig. 2a und 2b erkennbar ist, sind in der Wandung des Hauptkanals 6 zwei Öffnungen, Mündungen oder Bohrungen (im Folgenden der Einfachheit halber stets als Mündungen oder als Bohrungen bezeichnet, ungeachtet ihrer Herstellung) angeordnet, die Septumbohrung oder -mündung 7 und die Nebenkanalbohrung oder -mündung 8.

Um Bauraum zu sparen, sind diese beiden Bohrungen oder Mündungen in einigen erfindungsgemäßen Ausführungsformen so dicht wie möglich einander benachbart angeordnet.

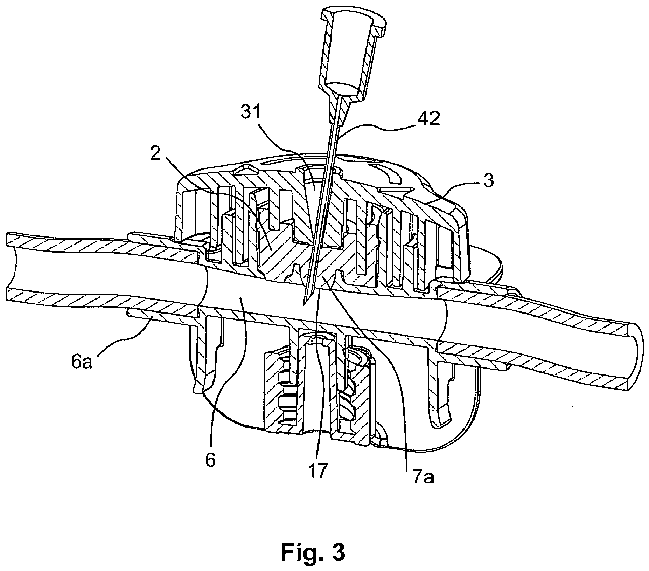

Die Septumbohrung 7 ist durch ein Septum 7a verschlossen. Ein Zugang zum Hauptkanal 6 ist mittels Durchstechen des Septums 7a, beispielsweise mithilfe einer durch den Einstichkanal 31 geführten Kanüle 42, möglich, wie dies in Fig. 3 dargestellt ist.
Die Nebenkanalbohrung 8 verbindet den Hauptkanal 6 mit weiteren Fluidsystemen, wie dies weiter unten beschrieben ist. Die Nebenkanalbohrung 8 mündet in den nicht mit Bezugszeichen markierten Nebenkanal, welcher Nebenkanäle 9 und 10 aufweist oder sich aus diesen zusammensetzt. Der Nebenkanal setzt sich im Nebenkanalrohr 11 fort und endet, soweit der Port 100 betroffen ist, im Nebenkanalkonnektor 12.

Sowohl die Septumbohrung 7 als auch die Nebenkanalbohrung 8 sind im Lumen oder in ihrer Öffnungsfläche so klein wie strömungstechnisch möglich ausgestaltet. Auf diese Weise stellen sie vorteilhaft sowohl im geschlossenen Ventilzustand (etwa bedingt durch Toleranzen des Dichtelements 2) als auch im geöffneten Ventilzustand eine möglichst geringe Beeinträchtigung der Strömung im Hauptkanal 6 dar.

Unabhängig hiervon sind die Septumbohrung 7 und/oder die Nebenkanalbohrung 8 des erfindungsgemäßen Ports 100 in einigen erfindungsgemäßen Ausführungsformen im Wesentlichen entlang einer Linie parallel zur Hauptströmungsachse, oder gemeinsam in einer Hälfte eines Querschnitts, oder in einer Hauptkanalschale des längs geschnittenen Hauptkanals 6 angeordnet. Auf diese Weise ist es vorteilhaft möglich, den Hauptkanal 6 bei anderen Ausführungsformen als den hier gezeigten beispielsweise als halboffenen Kanal mit festen Kanalwänden auszuführen. Dessen andere Hälfte kann aus herstell- oder anordnungstechnischen Gründen durch ein weiteres, hier ebenfalls nicht gezeigtes Gehäuseelement verkörpert werden. Das weitere Gehäuseelement kann in solchen Ausführungsformen aus einem anderen Material als das übrige Gehäuseelement, beispielsweise einer Folie, bestehen oder ein solches aufweisen. Münden oder befinden sich sowohl die Septumbohrung 7 als auch die Nebenkanalbohrung 8 wie in der hier gezeigten erfindungsgemäßen Ausführungsform gemeinsam auf einer Hauptkanal-Halbseite (im Längsschnitt) oder gemeinsam auf einer Querschnittshälfte, so erlauben sie dadurch vorteilhaft, im Dichtelement 2 eine Mehrzahl an Dichtfunktionen zusammenzufassen.

Erfindungsgemäß ist abweichend hiervon allerdings auch angedacht, die Septumbohrung 7 und Nebenkanalbohrung 8 - bezogen auf den Hauptkanal 6 oder auf die Hauptströmungsachse - einander gegenüberliegend anzuordnen. Vorteilhaft kann bei einer solchen Anordnung das Septum 7a für die Bedienperson unverändert gut erreichbar angeordnet sein, während die Nebenkanalbohrung 8 hinsichtlich anderer Aspekte im Port 100 angeordnet ist.

Der Hauptkanal 6 ist im Wesentlichen von zylindrischer oder prismatischer Querschnittsgrundform. In manchen erfindungsgemäßen Ausführungsformen weist der Hauptkanal 6 zumindest im Bereich der Septumbohrung 7 allerdings vorteilhafterweise eine abgeplattete Innenform 17, einen gerade verlaufenden Querschnittsabschnitt oder eine ebene Septumbohrung 7 auf (siehe Fig. 2a und 2b). Auf diese Weise kann das Dichtelement 2 mit einer ebenen Abdichtfläche formschlüssig und totraumfrei die Septumbohrung 7 gegen den Hauptkanal 6 abschließen, und zwar selbst dann, wenn durch Drehen des Betätigungselements 3 das Dichtelement 2 in eine andere Stellung als die geschlossene Stellung oder Ventilstellung übergeführt wird.

Hierdurch unterscheidet sich die Ausgestaltung der hier gezeigten erfindungsgemäßen Ausführungsform des Hauptkanals 6 beispielsweise von anderen, ebenfalls erfindungsgemäßen Ausführungsformen, in welchen der Hauptkanal 6 ausschließlich zylindrisch, jedenfalls aber im entsprechenden Querschnitt stets gekrümmt ausgestaltet ist. Bei einer solchen ausschließlich zylindrischen - oder auf andere Weise gekrümmten - Ausgestaltung würde das entsprechend ausgestaltete Septum 7a aufgrund seiner zylindrischen stirnseitigen Wölbung nach Drehen des Betätigungselements 3 nicht mehr bündig mit der auch im Bereich der Septumbohrung 7 zylindrischen Wandung des Hauptkanals 6 abdichten. Diese Nachteile werden durch eine flache Ausgestaltung eines Abschnitts, hier der abgeplatteten Innenform 17, des Hauptkanals 6 vorteilhaft vermieden.

In einigen erfindungsgemäßen Ausführungsformen beträgt der Durchmesser der Septumbohrung 7 weniger als die Hälfte des Durchmessers des Hauptkanals 6. Bei einer solchen Ausgestaltung kann man auch bei rein zylindrisch - oder anderweitig gekrümmt - ausgestaltetem Hauptkanal 6 eine stets ausreichende Abdichtung der Septumbohrung 7 durch das Septum 7a gewährleisten. Hierzu kann die hauptkanalseitige Verschlussstirn 18 des Dichtelements beispielsweise eine rotationssymmetrische, klöpperbodenartige Wölbung aufweisen. Bei einer solchen Ausgestaltung oder einer ähnlichen Ausgestaltung entstehen durch Drehung des Dichtelements 2 nur sehr geringe geometrische Unebenheiten beim Verschluss des Hauptkanals 6 gegenüber der Septumbohrung 7. Derartige Unzulänglichkeiten in der geometrischen Übereinstimmung bewegen sich in solchen Fällen im Bereich von weniger als 5 % des Durchmessers des Hauptkanals 6. Man erhält hiermit immer noch annähernd bündige und totraumfreie Abschlüsse der Septumbohrung 7 im Umfang des Hauptkanals 6. Somit kann vorteilhaft auf komplexe oder spritztechnisch aufwändige Gestaltungen des Hauptkanals 6 verzichtet werden.

Das Gehäuseelement 1 des in Fig. 2a und 2b gezeigten Ports 100 weist auf der dem Betätigungselement 3 gegenüberliegenden Seite des Hauptkanals 6 Befestigungselemente zum Befestigen oder Halten des Ports 100 auf. Zwei oder mehr Schnappzungen 38 und/oder biegenachgiebige Rippen aufweisende Wände oder Strukturen - als Beispiele für Befestigungselemente - ermöglichen die Aufnahme des Ports 100 z. B. an Sockeln oder anderen Halterungen einer Behandlungsvorrichtung. Da somit in der hier gezeigten erfindungsgemäßen Ausführungsform geeignete, beispielsweise federnde und/oder hinterschnittene Befestigungselemente bereits am Port 100 enthalten sind, brauchen solche Elemente, welche sich leicht abnützen und verschmutzen, vorteilhafterweise nicht an der Behandlungsvorrichtung, beispielsweise am Sockel, vorgesehen zu sein.

Am Sockel der Behandlungsvorrichtung oder an anderen Befestigungsorten für den Port 100 kann selbst dann vorteilhaft auf Hinterschnitte verzichtet werden, wenn am Port 100 Einsteckeinrichtungen oder dergleichen vorgesehen sind, welche nach Einstecken in ein Gegenstück des Sockels der Behandlungsmaschine eine aufgrund der zu überwindenden Reibkraft geeignet hohe Haltewirkung erzielen.

Fig. 2a und 2b zeigen ferner Abschnitte des Schalttopfs 15 sowie Bogenrippen 35, welche jeweils weiter unten detailliert beschrieben werden, jeweils im Schnitt.

In Fig. 3 ist dargestellt, wie mittels einer Kanüle 42, welche durch den Einstichkanal 31 geführt wird, das Septum 7a durchstoßen wird. Damit ist eine Fluidverbindung zwischen Kanüle 42 und Hauptkanal 6 hergestellt.

Das Septum 7a stellt zudem stets (durchstochen oder nicht) eine Abdichtung der Septumbohrung 7 gegenüber Fluidaustritt aus dem Hauptkanal 6, auch gegenüber der Kanüle 42, sicher. Diese Eigenschaft wird durch entsprechende Auswahl des Werkstoffs für das Septum 7a begünstigt. Vorzugsweise werden Isopren-, Chlorbutyl- und Brombutyl-Kautschuk-Mischungen verwendet. Diese Eigenschaft wird ferner durch eine geeignete Dicke oder Wandstärke des Septums 7a und durch Vorsehen einer dauerhaften Druckvorspannung des Septums 7a begünstigt. Die Druckvorspannung wird in der hier gezeigten erfindungsgemäßen Ausführungsform gewährleistet durch die beschriebenen biege- und scherelastischen Einspanngeometrien zwischen Gehäuseelement 1 und Betätigungselement 3.

Die geeignete Dicke oder Wandstärke des Septums 7a ist in einigen Ausführungsformen größer als die zweifache Länge des ovalförmigen Kanülenlumens, welches durch schwertförmiges Anschrägen zustande kommt, oder größer als der doppelte Durchmesser des runden Kanülenquerschnitts.

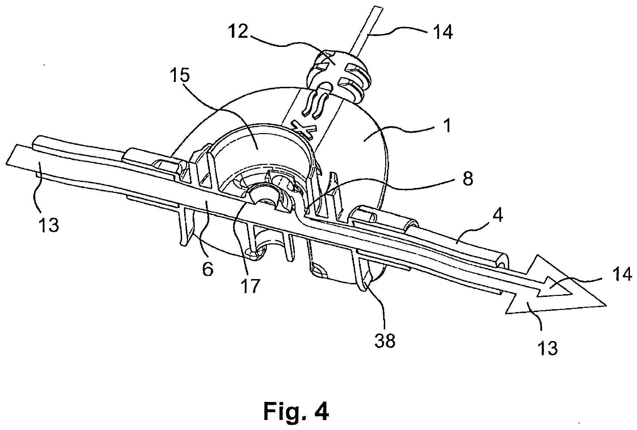

Fig. 4 zeigt die wesentlichen Fluidwege innerhalb des Ports 100 in einem Längsschnitt des Ports 100.

Eine Hauptkanalströmung 13 (großer Pfeil) durchströmt den Hauptkanal 6 von links nach rechts und stellt somit eine Fluidverbindung zwischen den beiden Anschlussschläuchen 4 her.

Eine Nebenkanalströmung 14 (kleiner Pfeil) strömt durch den Nebenkanalkonnektor 12 in das Nebenkanalrohr 11 ein und weiter über die Nebenkanalbohrung 8 in den Hauptkanal 6 ein. Gemeinsam mit der Hauptkanalströmung 13 verlässt die Nebenkanalströmung 14 den Port 100 über den in Fig. 4 rechts gelegenen Anschlussschlauch 4.

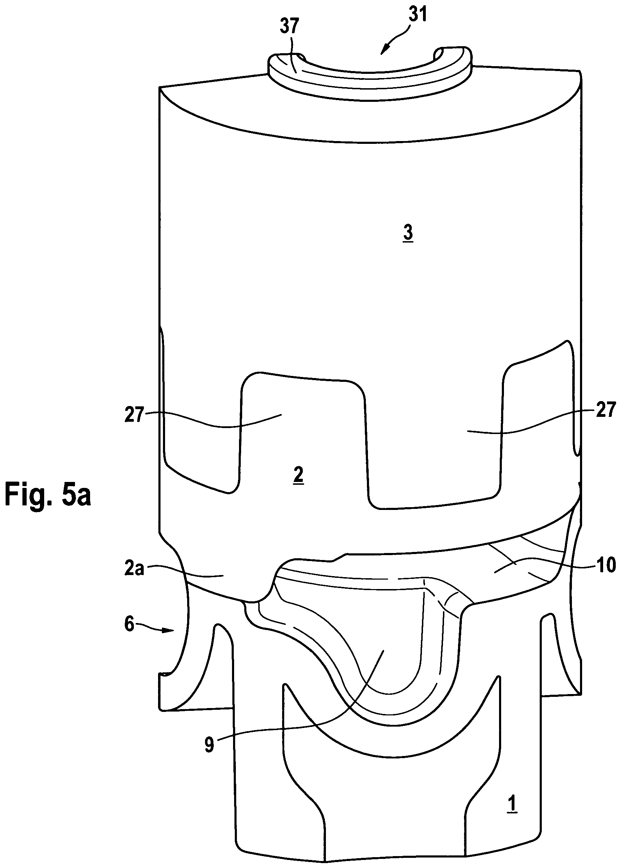

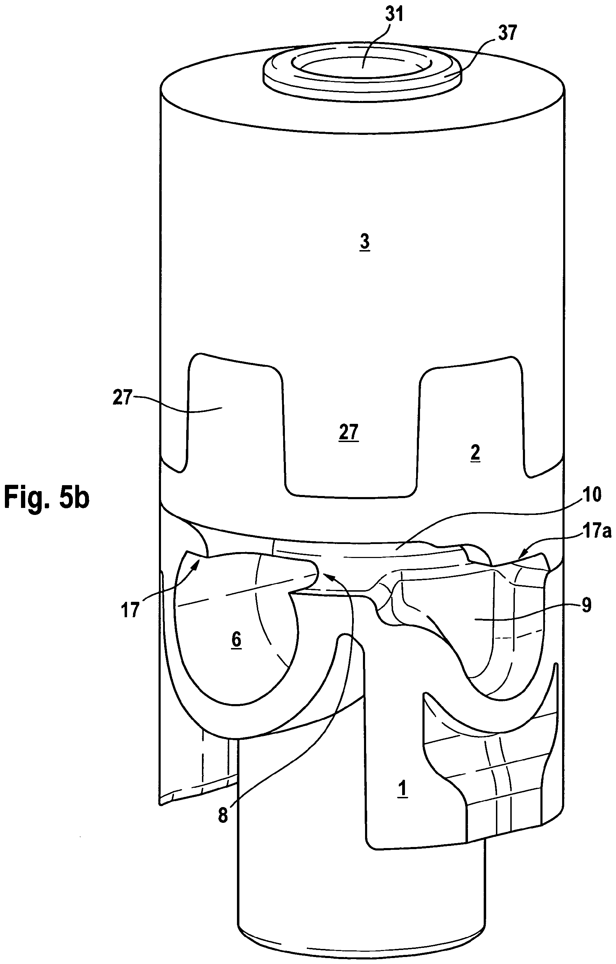

Die Fig. 5a und 5b zeigen Abschnitte des Ports 100, insbesondere des Betätigungselements 3 mit Drehmitnehmern 27, welche als Gabeln und Taschen ausgestaltet sind, in einem Peripheralschnitt ähnlichen einem aus dem Port 100 ausgestanzten Zylinder.

Dabei stellt Fig. 5a die geschlossene (Ventil-)Stellung, Fig. 5b die offene (Ventil-)Stellung dar. Die Darstellung der Fig. 5b ist gegenüber jener der Fig. 5a leicht gedreht.

Zu erkennen ist in beiden Figuren, dass der Nebenkanal, welcher seinen Ausgang an der Nebenkanalbohrung 8 nimmt und welcher den Hauptkanal 6 bei offener Ventilstellung über ein Nebenkanalrohr 11 und den Nebenkanalkonnektor 12 mit weiteren Fluidsystemen, wie dies weiter unten beschrieben ist, verbindet, zu einem Teil - als Nebenkanal 9 - im Gehäuseelement 1 verläuft, während er zu einem anderen Teil gleichzeitig - als Nebenkanal 10 - im Dichtelement 2 verläuft.

Zu erkennen ist in Fig. 5a ferner ein Abschnitt 2a des Dichtelements 2, welcher in der geschlossenen Ventilstellung dieser Figur eine Abdichtung des Nebenkanals (Bezugszeichen 9 und 10) gegenüber dem nur geschnitten angedeuteten Hauptkanal 6 bewirkt, indem er keinen Spalt zwischen Dichtelement 2 und der in Fig. 5a nicht gezeigten Nebenkanalbohrung 8 belässt. In Fig. 5b ist dieser Spalt zwischen Nebenkanalbohrung 8 und Dichtelement 2 gegeben, weshalb das Ventil geöffnet ist. Ein Fluid, welches vom rechten Rand der Fig. 5b beispielsweise in den Nebenkanal 9 des Gehäuseelements 1 einströmt, kann nun unter dem Dichtelement 2 hindurch zur freiliegenden Nebenkanalöffnung 8 hin und durch diese in den Hauptkanal 6 einströmen.

In Fig. 5b ist zu erkennen, dass der Hauptkanal 6 in seinem oberen Bereich eine Abplattung oder eine abgeplattete Innenform 17 (im Sinne eines gerade verlaufenden Abschnitts des Lumens des Hauptkanals 6) aufweist. Tatsächlich mündet die Nebenkanalbohrung 8 erkennbar zu einem Teil in die abgeplattete Innenform 17, zu einem anderen Teil in einen Bereich des Hauptkanals 6, in welchem dieser einen runden Querschnitt aufweist. Auch das Dichtelement 2 kann in einem Abschnitt hiervon einen Abplattung 17a aufweisen. Die Abplattung 17a kann bei geschlossener Nebenkanalbohrung 8 den oberen Bereich des Hauptkanals 6, in welchem dieser abgeplattet ist, stufenfrei verschließen.

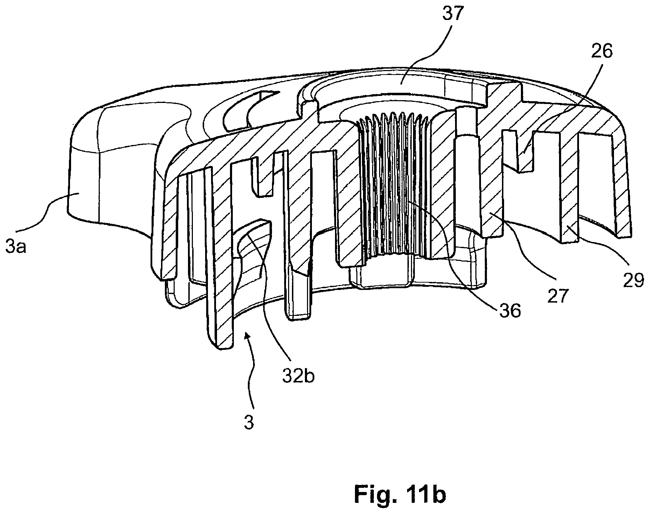

Zu erkennen ist in den Fig. 5a und 5b ferner ein Berührschutzsteg 37, welcher den Mündungsbereich des Einstichkanals 31 schützt. Beide Strukturen werden mit Blick auf Fig. 11b detailliert erläutert.

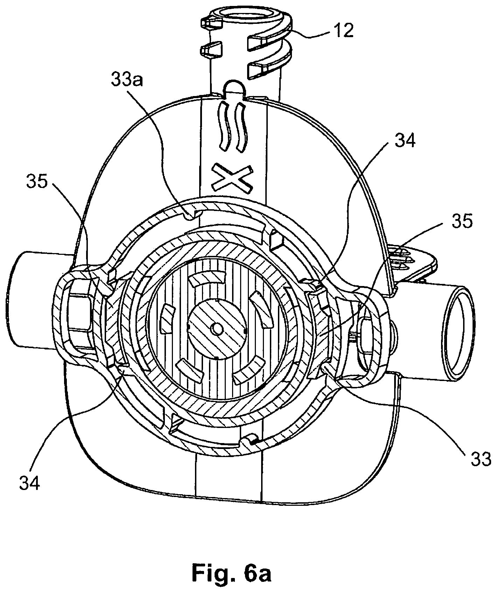

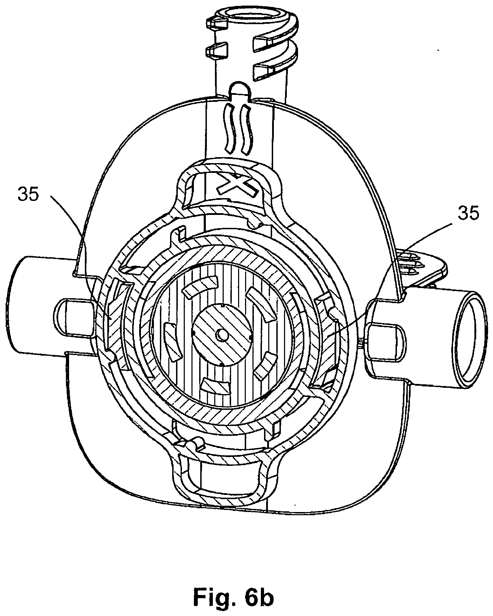

Fig. 6a und 6b zeigen den Port 100 in einem Frontalschnitt allein des Betätigungselements 3, in geschlossener Ventilstellung (Fig. 6a) sowie in offener Ventilstellung (Fig. 6b). Das Betätigungselement 3 steht in Fig. 6a in seiner zweiten Position, in Fig. 6b in seiner ersten Position. Beide Figuren zeigen, dass das drehbare Betätigungselement 3 Rastnasen 33a für eine Drehrastung 33 aufweist. Sie zeigen ferner, dass das Gehäuseelement 1 in zwei feststehenden Bogenrippen 35 Rastsenken 33b zur Aufnahme der Rastnasen 33a aufweist. Durch Einrasten einer ersten Rastnase 33a in einer ersten Rastsenke 33b wird eine erste Position des Betätigungselements 3 bis zu dessen erneuten Betätigen oder Drehen fixiert. Durch Einrasten der ersten Rastnase 33a in einer zweiten Rastsenke 33b (oder einer zweiten Rastnase 33a in der ersten Rastsenke 33b) wird eine zweite Position des Betätigungselements 3 bis auf weiteres fixiert. Dabei ist es unerheblich, ob das Betätigungselement 3 die Rastnasen 33a aufweist oder die Rastsenken 33b trägt.

Durch Drehanschläge oder Drehbewegungsanschläge 34 - wie sie in den Fig. 6a und 6b zu erkennen sind - oder anders ausgestaltete Anschläge können die Grenzen der Drehbarkeit des Betätigungselements 3 festgelegt sein. Derartige Drehanschläge 34 können in ihrer Bewegung, wie in Fig. 6a und 6b erkennbar, durch die Bogenrippen 35 begrenzt werden, beispielsweise von Kanten oder anderen Abschnitten der Bogenrippen 35.

Sowohl die Drehrastung 33 als auch die Drehanschläge 34 können dem Bediener eine taktile Rückmeldung über die Ventilstellung geben.

Da sowohl die Drehrastung 33 als auch die Drehanschläge 34 bevorzugt außenseitig, jedenfalls außerhalb der o. g. Fluidwege angeordnet sind, geraten sie mit keinem der Fluide in Kontakt. Mangels Kontakt mit Fluid geht von der Drehrastung 33 und den Drehanschlägen 34 selbst bei Materialabrieb keine Verunreinigung der Fluide aus. Die Drehrastung 33 und die Drehanschläge 34 können die fluidischen Toträume mangels Fluidkontakt auch nicht vergrößern. Zudem verschlechtern sie die Entlüftbarkeit des Ports 100 aus demselben Grund nicht.

Innerhalb des Schalttopfs 15 und im Bereich der Bogenrippen 35 entstehen Materialberührungen in bestimmten erfindungsgemäßen Ausführungsformen ausschließlich zwischen dem Gehäuseelement 1 und dem Dichtelement 2. Diese können in den berührenden und dichtverpressten Zonen jeweils dicht- und reibungsoptimiert sein. Im Fluidbereich bestehen in solchen Ausführungsformen somit keinen schleifenden Kontaktflächen zwischen Gehäuseelement 1 und Betätigungselement 3. Die Außenflächen des Schalttopfs 15 und entsprechende zugeordnete Innenflächen des Betätigungselements 3 übernehmen dagegen Aufgaben der spielfrei vorgespannten axialen und radialen Führung des Betätigungselements 3 gegen das Gehäuseelement 1. Hierbei können und dürfen Reibung und auch ein gewisser Abrieb entstehen. Durch die spielfreie Auslegung wird das Dichtelement 2 zentrisch und in optimaler Vorspannung zum Gehäuseelement 1 geführt. Verkippungen und Krafteinleitungen werden über das Betätigungselement 3 aufgenommen und damit nicht auf das Dichtelement 2 übertragen.

Die Bogenrippen 35, welche paarweise gegenüberliegend radial weiter außen angeordnet sein können, sind in ihrer Formgebung herstell- und funktionstechnisch optimiert. Die Bogenrippen 35 der Fig. 6a und 6b bilden, wie oben erwähnt, mit ihren Seitenflächen Begrenzungen für die Drehanschläge 34 der entsprechend angeordneten Radialrippen des Betätigungselements 3. Durch den gekrümmten Verlauf der Bogenrippen 35 ergeben sich hohe Steifigkeiten und Festigkeiten, wodurch die Bogenrippen 35 winkelpräzise und geschützt vor Überlastung zugleich sind. Durch die Bogengestalt in Verbindung mit der dünnen Wandstärke entsteht eine relativ dünne Rippe, welche im darunterliegenden Hauptkanal 6 spritzgießtechnisch optimiert geringe Einfallstellen verursacht.

Auf den beiden Bogenrippen 35 befinden sich die oben genannten Rastsenken 33b, welche die zugeordneten Rastnasen 33a des Betätigungselements 3 durch elastische Verformung sowohl der Bogenrippen 35 wie auch der Anbindungsstege der Rastnasen 33a durch Einrasten aufnehmen. Die Rastnasen 33a und die Rastsenken 33b sind so dimensioniert, dass vorzugsweise eine spielfreie Vorspannung in den gewünschten Grenzlagen des Betätigungselements 3 zustande kommt. Die Auslöse-Rastmomente sind so gewählt, dass der Anwender eine eindeutige taktile Rückmeldung über die sichere Betätigung erhält und darüber hinaus die gewünschte Ventilstellung mit ausreichender Genauigkeit und Selbsthaltung eingehalten wird. Die Überlastfestigkeit und die Schaltgenauigkeit sind umso günstiger, je weiter radial außen die entsprechenden Strukturen angeordnet werden. Ebenfalls aus Gründen der Überlastfestigkeit und der Schaltgenauigkeit sind die Drehanschläge 34 und die Rastnasen 33a bzw. die Rastsenken 33b paarweise punktsymmetrisch oder spiegelsymmetrisch zueinander vorgesehen. Die Kräfte werden so halbiert, während die Steifigkeiten verdoppelt werden. Zudem kompensieren sich die Kräfte als Kräftepaar zu einem reinen Drehmoment, wodurch Kipp- und Biegemomente zwischen Gehäuseelement 1 und Betätigungselement 3 vermieden werden. Bei Ausführungen mit erwünschten Dreh-Zwischenstellungen können optional die Bogenrippen 35 aufgeraute, fein verzahnte oder radial vorgespannte Bereiche aufweisen. Auch hiermit kann dem Anwender durch feine Rastungen oder durch erhöhtes Drehmoment mitgeteilt werden, in welchen Drehwinkelbereichen eine frei wählbare und selbsthaltende Zwischenstellung eingestellt werden kann.

Andererseits können die Drehwinkelbereiche zwischen den Rastpositionen auch bewusst reibungsarm gestaltet werden. Auf diese Weise kommt es nach der Überwindung einer Rastsenke 33b zu einer beschleunigten Drehbewegung des Betätigungselements 3, bis die nächste Rastsenke 33b erreicht wird. Auf diese Weise wird dem Anwender wiederum die Ventilstellung taktil mitgeteilt.

In weiteren erfindungsgemäßen Ausführungsformen wird der Drehwinkel zwischen zwei Funktionsstellungen bewusst derart klein gewählt, dass zwei benachbarte Rastsenken 33b ineinander übergehen, also in etwa eine Wellenform aufweisen. Auf diese Weise kann vermieden werden, dass beim Drehen des Betätigungselements 3 unerwünschte Zwischenstellungen eingenommen werden können.

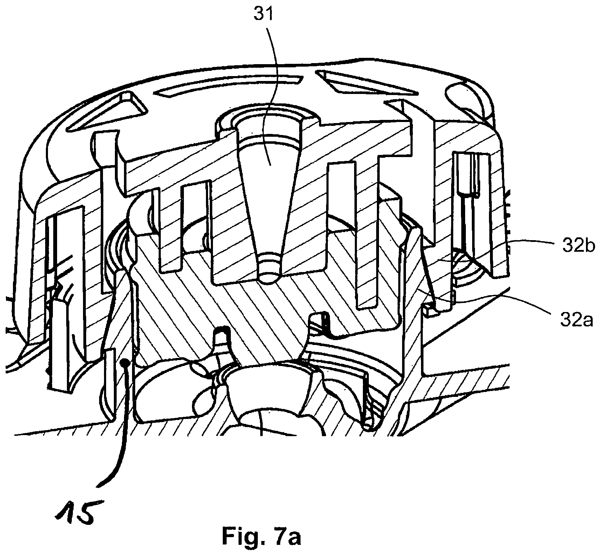

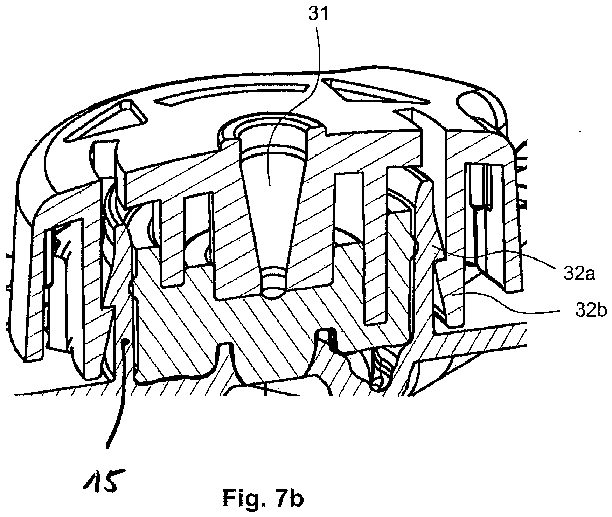

Wie in den Fig. 7a und 7b zu erkennen ist, weist der Schalttopf 15 - in einem beliebigen Bereich, vorzugsweise aber wie hier gezeigt in einem äußeren Bereich hiervon - in manchen erfindungsgemäßen Ausführungsformen eine oder mehrere Rastnasen 32a auf. Diese sind in bestimmten erfindungsgemäßen Ausführungsformen in geringer radialer Distanz zum Mittelpunkt des Schalttopfs 15 angeordnet.

Die Rastelemente 32a des Gehäuseelements 1 können mit Rastsenken 32b des Betätigungselements 3 verrastet werden. Die hierbei erzeugte Verrastung hält das Betätigungselement 3 am Schalttopf 15 und damit am Gehäuseelement 1. Für den Fachmann ist erkennbar, dass die Rastelemente 32a auch an anderer Stelle am Gehäuseelement 1 vorgesehen sein können als am Schalttopf 15.

Die Fig. 7a zeigt den Port 100 in einem Zustand, in welchem das Betätigungselement 3 (noch) nicht mit dem Gehäuseelement 1 verrastet ist. Fig. 7b zeigt den Port 100 der Fig. 7a in einem verrasteten Zustand.

Die Rastelemente 32a und 32b sind in einigen erfindungsgemäßen Ausführungsformen zur unlösbaren und spielfreien Verbindung zwischen Gehäuseelement 1 und Betätigungselement 3 als im Querschnitt pfeilförmige, komplementäre und radial ganz oder nur abschnittsweise umlaufende, d. h. in einem Querschnitt geschlossene, Strukturen ausgestaltet.

Die Rastelemente 32a und 32b sind in manchen erfindungsgemäßen Ausführungsformen ähnlich der Rillenverzahnung von Schlauchtüllen ausgestaltet.

Die Rastelemente 32a und 32b dienen dem Zusammenfügen von Gehäuseelement 1 und Betätigungselement 3. Sie können auch als Schnappelemente ausgestaltet sein.

Die Rastelemente 32a und 32b erlauben in manchen erfindungsgemäßen Ausführungsformen, wie der in den Figuren gezeigten, eine Drehbewegung zwischen Gehäuseelement 1 und Betätigungselement 3.

Zum Verrasten der Rastelemente 32a und 32b sind aufgrund ihrer (im Querschnitt) rampenförmigen Flächen vorteilhaft nur geringe Einbaukräfte in Einbaurichtung erforderlich. In Ausbaurichtung verhindern sie dennoch über im Wesentlichen senkrecht zur Verrastrichtung angeordnete Flächen in gewissen erfindungsgemäßen Ausführungsformen jedes zerstörungsfreies Entrasten. Im Vergleich zu freistehenden Schnappzungen sind die zylindrisch umlaufenden Schnappstrukturen bei geringem Materialeinsatz deutlich steifer und fester. Dadurch lassen sich selbst bei preisgünstigen unverstärkten Thermoplasten und auch bei Wandstärken im Zehntelmillimeterbereich günstige Einbaukräfte im Bereich von 50 N realisieren, welche mit vorteilhaft hohen Ausbau- oder Zerstörungskräften im Bereich von mehr als 500 N einhergehen.

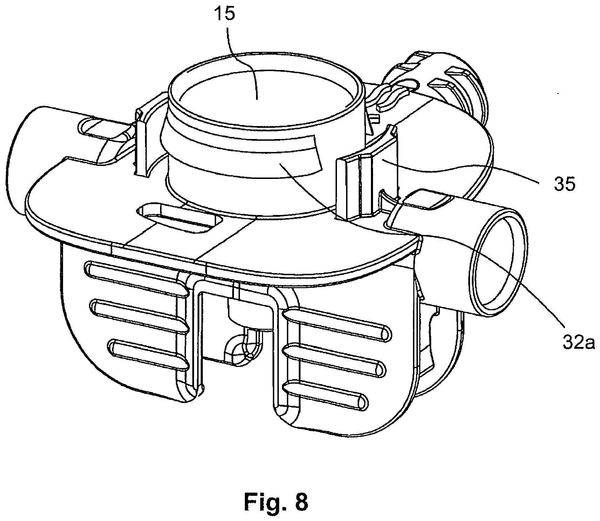

Fig. 8 zeigt das Gehäuseelement 1 der ersten Ausführungsform der Fig. 1a und 1b in perspektivischer Ansicht. Gut zu erkennen ist der Schalttopf 15. Ebenfalls gut zu erkennen sind beidseits des Schalttopfs 15 angeordnete Bogenrippen 35. Diese dienen als Begrenzung einer Drehbewegung der Drehanschläge 34, wie oben erläutert.

Bei der hier dargestellten Gestaltung sowohl des Gehäuseelements 1 als auch des Betätigungselements 3 können diese unter Anwendung besonders wirtschaftlicher Entformungsmethoden mittels Spritzguss hergestellt werden. Zum Herstellen des Gehäuseelements 1 genügt als Werkzeug eine Standardkombination aus Backen und Kernen, während beim Betätigungselement 3 sogar ein sogenanntes Auf-Zu-Werkzeug ausreicht. Diese Entformungsmethoden führen dazu, dass sowohl das Gehäuseelement 1 als auch das Betätigungselement 3 jeweils ein, zwei oder mehr Unterbrechungen der ansonsten umlaufenden Rast- oder Schnappstruktur aufweisen können. Die Winkelstellungen und Winkelbereiche der verbleibenden Raststrukturen beider Elemente sind in diesen Fällen so festgelegt, dass es in der bevorzugten Position des Betätigungselements 3 zu einer maximalen, jedenfalls aber ausreichenden Überlappung der beiden Rastelemente 32a und 32b kommt. Aber auch in allen anderen möglichen Schaltstellungen ist stets für eine ausreichende Überlappung gesorgt. In bestimmten erfindungsgemäßen Ausführungsformen sind alle oder einige der Rastelemente 32a einerseits und/oder der Rastelemente 32b andererseits paarweise (z. B. spiegelbildlich oder achsensymmetrisch bezogen auf einen Drehmittelpunkt des Betätigungselements 3) gegenüberliegend angeordnet. Auf diese Weise kommt es bei einer gegebenen, auf die Verrastung wirkenden axialen Vorspannung nicht zu einer hieraus resultierenden, unerwünschten Verkippung einzelner Elemente.

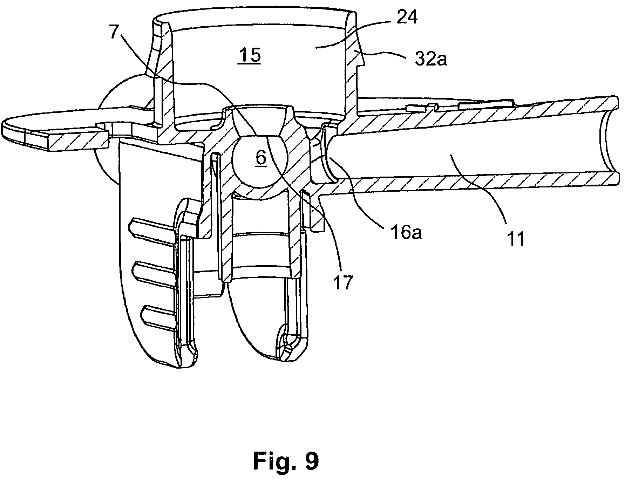

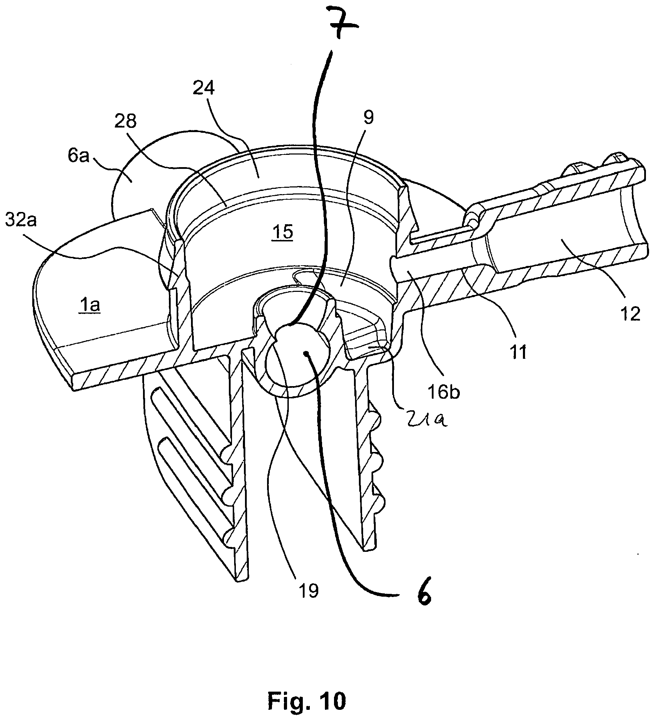

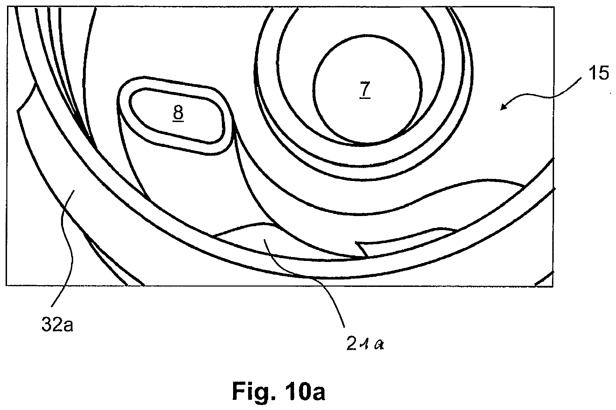

Fig. 9 zeigt das Gehäuseelement 1 des Ports aus Fig. 1a und 1b in leichter Perspektive im Längsschnitt durch das Nebenkanalrohr 11 des Nebenkanals. Gut zu erkennen ist eine Stopfbuchs-Dichtzone 24 des Schalttopfs 15 (siehe auch Fig. 10).

Das Nebenkanalrohr 11 steht über eine Nebenkanalbohrung 16a des Schalttopfs 15 in Fluidverbindung mit dem Hauptkanal 6.

Fig. 10 zeigt das Gehäuseelement 1 einer weiteren Ausführungsform in einem Längsschnitt durch den Nebenkanal, wiederum in perspektivischer Ansicht.

Das Nebenkanalrohr 11 steht über eine Nebenkanalbohrung 16b des Schalttopfs 15 einer weiteren Ausführungsform in Fluidverbindung mit dem Hauptkanal 6.

Wie in Fig. 10 gezeigt ist, kann der Hauptkanal 6 im Bereich der Septumbohrung 7 - und/oder im Bereich der Nebenkanalbohrung 8, obgleich hier nicht gezeigt - vorzugsweise ebenfalls eine abgeplattete Innenform 17 aufweisen. Ergänzend oder alternativ hierzu kann der Hauptkanal 6 einen Formwechsel 19 oder Übergang von abgeplatteter Form zu im Wesentlichen zylindrischer Form des Umfangs des Lumenquerschnitts aufweisen.

In bestimmten erfindungsgemäßen Ausführungsformen befinden sich die Septumbohrung 7 und/oder die Nebenkanalbohrung 8 zu je etwa gleich großen Anteilen hiervon im Bereich der abgeplatteten Hauptkanalform und im Bereich der zylindrischen Hauptkanalform. Die Septumbohrung 7, oder die Nebenkanalbohrung 8, durchschneidet also die durch Zusammentreffen der gekrümmten oder zylindrischen Abschnitte des Hauptkanalquerschnitts mit dessen abgeplatteten oder geraden Abschnitten gebildete, enger verrundete Kante. Auf diese Weise ist es bezogen auf die Nebenkanalbohrung 8 vorteilhaft möglich, einerseits durch Drehen des Dichtelements 2 einen hinreichend großen Öffnungsquerschnitt der Nebenkanalbohrung 8 freizugeben oder zu verschließen, wobei hierzu nur eine geringe Drehbewegung erforderlich ist. Andererseits berühren sich Gehäuseelement 1 und Dichtelement 2 im Bereich der dichtenden Bohrungswände nur geringfügig; sie haben einen nur geringen Drehkontakt miteinander. Dies minimiert störendes Bewegungsreiben und eine sich hieraus ggf. ergebende verminderte Dichtpressung und/oder ein Zurückbleiben der die Öffnungen des Hauptkanals 6 abdichtenden Abschnitte des Dichtelements 2 relativ zum nominellen Schaltwinkel, oder verhindert dies vollständig.

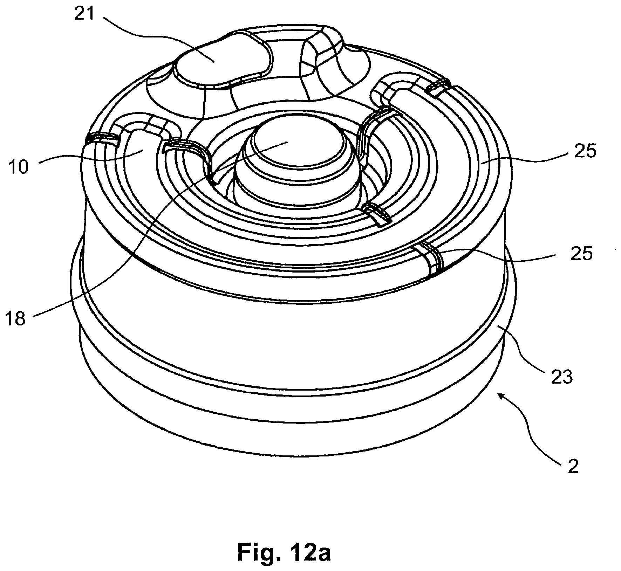

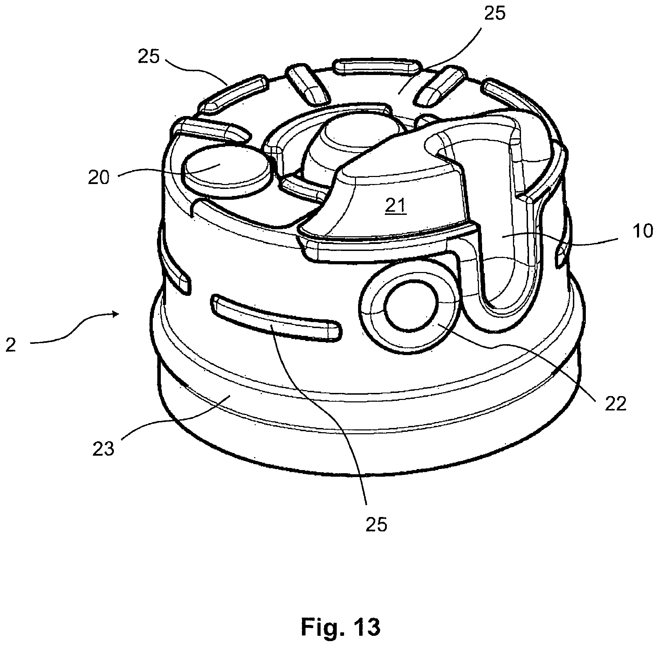

Fig. 10a zeigt bei einem Blick von schräg oben in einen Ausschnitt des Schalttopfs 15 eine Vertiefung 21a im Nebenkanal 9, in welcher sich eine Dichtnase 21, siehe Fig. 12a und 13, verdrehen lässt.

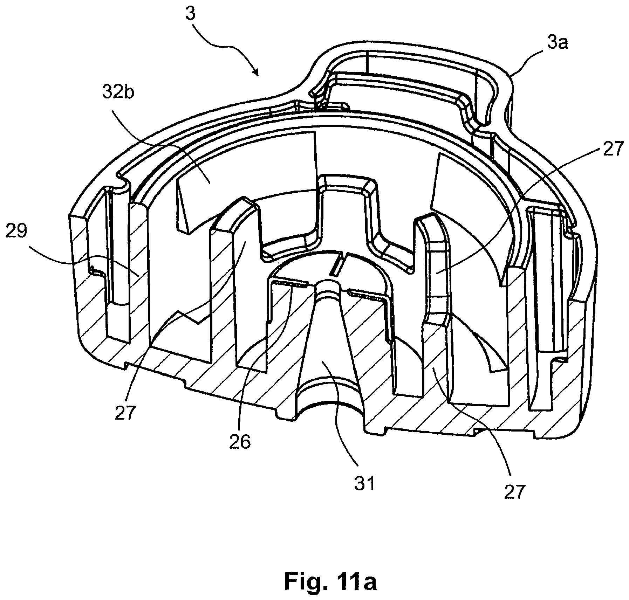

Fig. 11a zeigt die Unterseite des Betätigungselements 3 einer weiteren Ausführungsform in einem Querschnitt durch den Einstichkanal 31 hindurch, wiederum in perspektivischer Ansicht.

Zur Gewährleistung des Berührschutzes der Oberfläche des Septums 7a ist diese nur über einen Einstichkanal 31 zu erreichen. Der Einstichkanal 31 kann gemäß Fig. 11a derart eng ausgestaltet werden, dass es selbst bei maximaler Verkippung der Achse der Kanüle 42 zur Achse des Einstichkanals 31 nicht möglich ist, mit der Spitze der Kanüle 42 in Materialbereiche des Gehäuseelements 1 einzustechen, welche um die Septumbohrung 7 herum liegen und welche den Hauptkanal 6 begrenzen. Damit ist ein Durchstechen des Septums 7a besonders ergonomisch und bei hoher Sicherheit vor einer Kontamination des Fluids des Hauptkanals 6 - durch Materialablösung und - verschleppung mittels Kanüle 42 - möglich. Der Zugang zur Oberfläche des Septums 7a mittels Sprühdesinfektionsmitteln bleibt gewährleistet.

Das Betätigungselement 3 weist ferner als Stege und Nuten ausgestaltete Elemente 26 auf. Diese begünstigen die Sterilisierbarkeit und die Kompressibilität, ferner stützen sie.

Zu erkennen sind in Fig. 11a ferner wiederum die als Taschen und Gabeln ausgestalten Drehmitnehmer 27.

Fig. 11b zeigt das Betätigungselement 3 einer wiederum weiteren Ausführungsform in einem Querschnitt durch den Einstichkanal 31 hindurch, wiederum in perspektivischer Darstellung, mit Blick auf die Oberseite.

Um eine erneute Trocknung oder Rücktrocknung der Oberfläche des Septums 7a nach Sterilisierung mittels Fluid zu beschleunigen, weist die innere Wand des Einstichkanals 31 in manchen erfindungsgemäßen Ausführungsformen eine Rillenstruktur 36 auf, wie Fig. 11b zeigt. Die Rillenstruktur 36 kann bis an die Oberfläche des Septums 7a heranreichen. Die Rillen der Rillenstruktur 36 sind hinreichend tief und eng zueinander ausgestaltet mit zahlreichen Vertiefungen oder Kapillaren zum Aufnehmen eines flüssigen Desinfektionsmittels (beispielsweise mittels Adhäsion) und um dieses in Richtung des offenen Ausgangs des Einstichkanals 31 erneut abzuleiten. Auf diese Weise entsteht sowohl eine schnelle Ausdünnung und/oder Verteilung des flüssigen Desinfektionsmittels am Septum 7a als auch eine beschleunigte Verdunstung aufgrund der erzielten Oberflächenvergrößerung.

Der Einstichkanal 31 kann weiterhin vorteilhaft so gestaltet sein, dass er sich zu beiden Seiten hin aufweitet. Der Einstichkanal 31 kann trichterförmig sein. Seine engste Stelle liegt der Oberfläche des Septums 7a zugewandt. Die engste Stelle liegt in einigen erfindungsgemäßen Ausführungsformen etwa um die Länge einer Kanülenschneide von der Oberfläche des Septums 7a entfernt. Während die Aufweitung im Bereich der Einstichmündung dem erleichterten Einführen der Kanüle 42 in die Mündung dient, soll die zweite - in der Regel geringere - Aufweitung am anderen Ende des Einstichkanals 31 verhindern, dass die Kanüle 42 an der Wandung des Einstichkanals 31 einen Span oder Partikel ablöst, welcher mit der Spitze der Kanüle 42 durch das Septum 7a in den Hauptkanal 6 hineinverschleppt werden könnte. Die Spitze der Kanüle 42 gelangt so nach dem Passieren der engsten Stelle noch einmal "ins Freie". Dies senkt die Wahrscheinlichkeit, dass die Spitze der Kanüle 42 in die ringförmig umlaufende Kante zwischen Oberfläche des Septums 7a und der Wandung des Einstichkanals 31 eingestochen wird. Damit der enge Einstichkanal 31 nicht zu einem erschwerten Einführen Kanüle 42 führt, kann sich dieser zu einem größeren Durchmesser weiten; dabei bleiben die Vorteile, die mit der Begrenzung der möglichen Einstichwinkel erzielbar sind, erhalten.

Fig. 11b zeigt einen weiteren umlaufenden Berührschutzsteg 37 von größerem Durchmesser. Auch er schützt den Mündungsbereich des eigentlichen Einstichkanals 31 vor einer Berührung. Insbesondere kann mittels des Berührschutzstegs 37 verhindert werden, dass der Anwender das Gehäuse im Bereich der Einstichmündung berührt, beim Einstechen die Mündung nicht trifft und/oder die unsterilen Bereiche mit der Kanüle 42 berührt und schließlich mit der kontaminierten Kanüle 42 anschließend das Septum 7a durchsticht. Damit ist ein Infektionsschutz selbst für den Fall vorteilhaft noch gewährleistet, dass der Anwender auf eine Flüssigdesinfektion verzichten sollte.

Der Port 100 ist in bestimmten erfindungsgemäßen Ausführungsformen für jede bekannte Sterilisationsart, insbesondere solche, bei welchen Strahlung, Gas oder Dampf zum Einsatz kommen, geeignet.

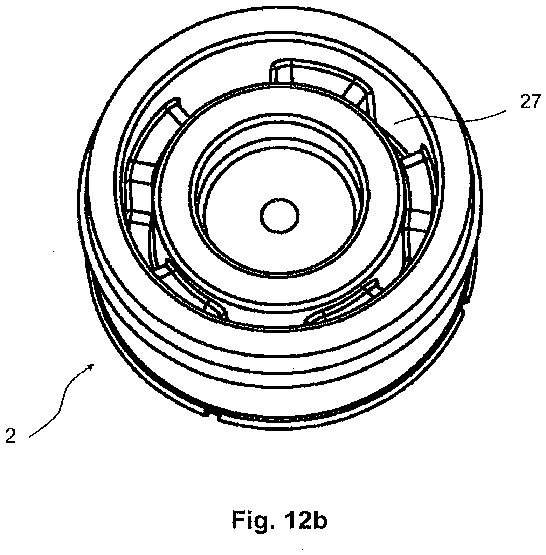

Für Sterilisationsarten, bei welchen sterilisierende Fluide alle relevanten Oberflächen erreichen müssen, sind geeignete Diffusionswege zwischen benachbarten oder einander berührenden Oberflächen vorzusehen. Wie mit Details bereits zuvor beschrieben, weist ein Großteil aller Oberflächen, an welchen sich das Gehäuseelement 1, das Dichtelement 2 und/oder das Betätigungselement 3 berühren können, zahlreiche erhabene oder vertiefte Stütz-, Drainage-, und Kompressibilitätsstrukturen 25 auf. Solche Strukturen sind in Fig. 12a, welche das Dichtelement 2 von der Seite seines Kontakts mit dem Fluid zeigt, und in Fig. 12b, welche das Dichtelemente 2 von der Seite des Betätigungselements 3 aus betrachtet zeigt, zu erkennen. Diese Strukturen 25 sind in bestimmten erfindungsgemäßen Ausführungsformen zugleich hinreichend nachgiebig, steif und in optimiertem Teilungsabstand unter optimierten Freiraum-Spaltmaßen angeordnet. Einige dieser Stege können bogenförmig ausgestaltet sein. Dies kann vorteilhaft dazu beitragen, ein Bewegungsreiben zu minimieren.

In einigen erfindungsgemäßen Ausführungsformen befindet sich die Nebenkanalbohrung 8 vollständig im Bereich der abgeplatteten Innen- oder Umfangsfläche des Hauptkanals 6. Diese Ausführungsform eignet sich besonders gut für eine Drehbewegung des Dichtelements 2, kombiniert mit einer im Wesentlichen auf axialen Verpresskräften beruhenden Abdichtung der Nebenkanalbohrung 8. Bei einer solchen axial abdichtenden Ausführung weist das Dichtelement 2 beispielsweise eine stirnseitige Abdichtfläche 20 auf (siehe Fig. 13). Die stirnseitige Abdichtfläche 20 steht - unter im Wesentlichen konstant bleibender axialer Vorspannung - in der geschlossenen Ventilstellung zentrisch abdichtend oder gar überlappend über der Nebenkanalbohrung 8. Die stirnseitige Abdichtfläche 20 gelangt, wenn das Dichtelement 2 mittels des Betätigungselements 3 zur geöffneten Ventilstellung hin gedreht wird, in einen Bereich neben der Nebenkanalbohrung 8. Ein direkt neben der Abdichtfläche 20 platzierter, im Dichtelement 2 des Nebenkanals 10 liegender Abschnitt kommt, wenn das Betätigungselement 3 zum Öffnen des Ventils gedreht wird, über der Nebenkanalbohrung 8 zu liegen und leitet einen Fluidstrom zu weiteren Nebenkanalstrukturen des Schalttopfes 15 über. Da die stirnseitige Abdichtfläche 20 im Gebrauch im Schalttopf 15 bei dieser Ausführungsform zur Gewährleistung der konstanten Dichtverpressung im Wesentlichen eben und senkrecht zur Drehbetätigungsachse angeordnet ist, ist in solchen Ausführungsformen auch der Hauptkanal 6 an der Stelle der Nebenkanalbohrung 8 am stärksten abgeplattet. Auf diese Weise besteht die Möglichkeit, eine gleichmäßige dünne Wandung zwischen Hauptkanal 6 und Stirn des Schalttopfs 15 vorzusehen. Die Wandung ist in einigen erfindungsgemäßen Ausführungsformen nur etwa maximal 10 % des Hauptkanaldurchmessers stark. Hierdurch bildet sich in der geschlossenen Ventilstellung ein entsprechend geringes Sackloch am Hauptkanal 6 aus, was vorteilhaft Totraum vermeidet.

In bestimmten erfindungsgemäßen Ausführungsformen, beispielsweise in der oben beschriebenen ersten Ausführungsform, weist das Dichtelement 2 einen oder mehrere Stopfbuchs-Dichtringe 23 auf, wie in Fig. 13 zu erkennen ist. Stopfbuchs-Dichtringe 23 des Dichtelements 2 können an Stopfbuchs-Dichtzonen 24 des Schalttopfs 15, siehe Fig. 9 und 10, anliegen. Stopfbuchs-Dichtringe 13 können kreisförmig das Dichtelement 2 umgeben.

Zur Steigerung der Dichtwirkung kann das Dichtelement 2 in seinem Inneren in radialer Richtung durch - beispielsweise ringförmige - Stege des Bestätigungselements 3, und/oder an seinem Äußeren durch - beispielsweise ringförmige - Elemente des Schalttopfs 15 abgestützt und verpresst werden. Derartige Stege, Elemente oder dergleichen werden im Folgenden auch als Stützstrukturen bezeichnet.

In manchen Figuren und vor allen in Fig. 10 erkennt man, dass der Schalttopf 15 zusätzlich zu seinem zylindrischen Innenbereich, welcher ebenfalls das Dichtelement 2 stützt, eine weitere Stützstruktur in Gestalt eines - beispielsweise gestuften - Bereichs 28 aufweist. Der gestufte Bereich 28 weist einen Abschnitt mit einem mehr oder weniger kegelförmigen oder zulaufenden Querschnitt oder eine umlaufende Stufe auf. Je nach axialer Anordnung des Dichtelements 2 - bezogen darauf, wie tief es in den Schalttopf 15 eingesteckt ist - lässt sich somit wahlweise eine rein radiale Verpressung zwischen den rein zylindrischen Stützstrukturen des Schalttopfs 15 erzielen, oder eine halbaxiale oder eine rein axiale Dichtverpressung an dem mehr oder weniger kegelförmig oder zulaufend ausgestalteten Abschnitt des gestuften Bereichs 28.

Damit das Dichtelement 2 wegen seiner permanenten Verpressung nicht in die noch nicht abgestützte axiale Raumrichtung zu kriechen beginnt, vor allem in Richtung des offenen Endes des Septumtopfs, weist das Betätigungselement 3 vorzugsweise eine beispielsweise ringförmig ausgestaltete Axialstützrippe 29 auf. Diese drückt auf die nach außen weisende Stirnfläche des Dichtelements 2 und führt zu einer elastisch wirkenden axialen Vorspannung bis in den Bereich des Stopfbuchs-Dichtrings 23 hinein. Axiale und radiale elastische Verformungen kommen dadurch zustande, dass nicht das gesamte Volumen des Dichtelements 2 durch das Gehäuseelement 1 und/oder durch das Betätigungselement 3 begrenzt ist. Vielmehr gibt es Bereiche, in denen das Dichtelement 2 in Kontakt steht mit Flüssigkeit oder Gas. Auf diese Weise wird dem Dichtelement 2 eine elastische Biege- und Scherbewegung zugestanden. Die speziell austarierte wechselnde Anordnung von vorgespannt abgestützten und nicht abgestützten Bereichen sorgt für eine gleichbleibende, ermüdungsfreie und toleranzentolerante Vorspannung des Stopfbuchs-Dichtrings 23 und damit für eine dauerhafte Dichtheit bei Herstellmontage, Sterilisation, Lagerung und Gebrauchseinsatz.

Das Septum 7a und die es umgebenden Abschnitte des Dichtelements 2 gewährleisten aufgrund ihrer Form die erforderliche Abdichtung zur Septumbohrung 7 im Gehäuseelement 1 bzw. die erforderliche Abstützung des Dichtelements 2 gegenüber dem Betätigungselement 3. Die Gestaltung der Verschlussstirn 18 des Septums 7a wurde bereits im Hinblick auf den totraumfreien Abschluss der Septumbohrung 7 in allen Stellungen des Betätigungselements 3 beschrieben. Die bombierten oder sich verjüngenden Seitenflächen 30 (Fig. 2a) und die zugeordneten, mit anderer Krümmung bombierten Seitenflächen des Dichtbereiches des Septums zur Septumbohrung 7 hin sind rotationssymmetrisch zur Drehachse des Betätigungselements 3 ausgestaltet. Die bombierten Seitenflächen 30 führen zu einer gemischt axial-radialen Dichtweise unter axialer Vorspannung. Durch die unterschiedlichen Bombierungen der Seitenflächen 30 mit anliegenden und freien Zonen kommt es wieder zu den oben beschriebenen Effekten einer optimalen elastischen Vorspannung. Als weitere vorteilhafte Effekte dieser Gestaltungsweise sind zu nennen: a) beim Zusammenbau ergibt sich eine selbsttätige Zentrierung, b) die freien Räume stehen durch Sterilisationsnuten 25 mit weiteren freien Räumen in gasdurchlässiger Verbindung, so dass die Sterilisation vorteilhaft durch direkten Stoffaustausch gefördert wird und keine langen Diffusionswege für das Sterilisationsmedium entstehen, c) es entsteht zusätzlich eine permanente Druckspannung im Septummaterial, welche die Wiederabdichtung des Stechkanals nach dem Herausziehen der eingestochenen Kanäle vorteilhaft fördert, d) zu große Reibedrehmomente, welche häufig durch zu große verpresste Elastomeroberflächen verursacht werden, werden vermieden, und damit ungünstige Betätigungsmomente ebenso wie Torsionsverformungen des Elastomers, welche durch Gestaltänderungen zu Undichtheiten führen können.

Die dem Betätigungselement 3 zugewandten Abschnitte des Dichtelements 2 sind überwiegend so gestaltet, dass - vorzugsweise im Wesentlichen oder vorwiegend - eine vollflächige Abstützung des Dichtelements 2 zur Verhinderung von Torsion, Axialverschiebung und radialem Kriechen gewährleistet ist. Das Betätigungselement 3 hat an seiner dem Dichtelement 2 zugewandten Unterseite zu diesem Zweck bevorzugt drei ringförmige Ringstege, welche in entsprechende Nuten des Dichtelements 2 passgenau, bevorzugt sogar unter räumlicher Aufweitvorspannung, eingreifen. Der innerste Ringsteg bildet zugleich den Kanülen-Einstichkanal 31, der mittlere umfasst zugleich die Drehmitnehmer 27 und der äußere zugleich die Axialstützrippe 29. Der innere Ringsteg weist stirnseitig bevorzugt feine Nuten oder Stege 26 auf, welche die sichere Sterilisation der Randzone der Einstichzone der Kanüle 42 gewährleisten, ohne die axiale Abstützung einzuschränken.

Die Abdichtung der Nebenkanalbohrung 8 gegenüber dem Hauptkanal 6 und gegenüber den Nebenkanalstrukturen lässt sich in einigen erfindungsgemäßen Ausführungsformen wie folgt erzielen:
Der Schalttopf 15 weist - vorwiegend im Bereich seiner Stirnflächen in Nähe des Hauptkanals 6 - Nebenkanalstrukturen auf, welche als Nebenkanal 9 des Gehäuseelements 1 bezeichnet werden. Sie dienen hauptsächlich für eine fluidische Verbindung zwischen der Nebenkanalbohrung 8 im Hauptkanal 6 und der Nebenkanalbohrung 16 im Schalttopf 15 (siehe Fig. 10).

Je nach Ausführungsform und Schaltstellung können sich die Strukturen des Nebenkanals 9 im Gehäuseelement 1 mit zugeordneten Strukturen des Nebenkanals 10 des Dichtelements 2 ergänzen. Gemeinsam leiten die Strukturen des Nebenkanals 9 und die Strukturen des Nebenkanals 10 das Fluid, welches die Nebenkanalströmung 14 ausmacht.

Zudem bilden die Wandungen der Nebenkanäle 9 und 10, insbesondere in unmittelbarer Nachbarschaft zu den Bohrungen zum Hauptkanal 6, in einigen erfindungsgemäßen Ausführungsformen einen oder mehrere Dichtsitze des Dichtelements 2. Bedingt durch ihre Form und durch die Form des Dichtelements 2 erlauben diese Strukturen beispielsweise eine Drehung des Dichtelements 2, welche mit zumindest einer Endlage winkelbegrenzt ist. Dabei kann es in der Endlage der Drehung zu einer Abdichtung bzw. zu einem totraumfreien Abschluss der Septumbohrung 7 und/oder der Nebenkanalbohrung 8 gegenüber dem Hauptkanal 6 kommen.

Auch in beliebigen Zwischenstellungen, welche das Dichtelement 2 bei seiner Drehung innerhalb des Gehäuseelements 1 einnehmen kann, sorgen die im Wesentlichen zylindrischen Wandungsstrukturen in Verbindung mit dem Stopfbuchs-Dichtring 23 des Dichtelements 2 (siehe Fig. 12a oder 13) und der Stopfbuchs-Dichtzone 24 des Schalttopfs 15 (siehe Fig. 10) für eine permanente Fluidabdichtung nach außen.

Um Schaltbewegungen zu erlauben und dabei gleichzeitig Toträume nach Möglichkeit zu vermeiden, ist beispielsweise eine Dichtnase 21 (Fig. 12a) des Dichtelements 2 in bestimmten erfindungsgemäßen Ausführungsformen angeordnet. Diese kann beim Übergang von der geschlossenen in die offene Ventilstellung bewegt werden, beispielsweise in einer Drehbewegung oder auf einer Drehkurve um einen Drehpunkt. In manchen dieser Ausführungsformen ist im Gehäuseelement 1 ein freier Mindestraum oder eine Vertiefung 21a, siehe Fig. 10a, zur Beherbergung der Dichtnase 21 vorgesehen. Der freie Mindestraum ist in einigen erfindungsgemäßen Ausführungsformen aufgrund der Ausgestaltung des Nebenkanals 9 im Schalttopf 15 vorgesehen, wie aus den unterschiedlichen Ausgestaltungen der Fig. 9, 10 und 10a zu erkennen ist.

Je nach räumlicher Lage der Nebenkanalbohrung 8 im Schalttopf 15, siehe für Varianten die Fig. 9 und 10, und je nach Weite des Dreh- oder Schaltwinkelbereichs des Betätigungselements 3 und der Anordnung der Nebenkanalbohrung 8 zum Hauptkanal 6, können radiale und axiale Strukturen sowohl des Nebenkanals 9 des Gehäuseelements 1 als auch des Nebenkanals 10 des Dichtelements 2 unterschiedlich, und vor allem verschieden lang, ausgestaltet sein. So weist das Dichtelement 2 nach Fig. 12a überwiegend radial am Dichtelement 2 ausgestaltete Strukturen des Nebenkanals 10 auf, während der Nebenkanal 10 des Dichtelements 2 nach Fig. 13 überwiegend axiale Strukturen - bezogen auf das Dichtelement 2 - aufweist.

Die Ausgestaltungen des Gehäuseelements 1 nach Fig. 9 und des Dichtelements 2 nach Fig. 12a weisen gemeinsam nur eine schaltbare Abdichtung oder Unterbindung der Nebenkanalströmung 14 gegenüber der Hauptkanalströmung 13 auf. Diese erfolgt am Übergang der Nebenkanalbohrung 8 zum Hauptkanal 6. Die schaltbare Anordnung ermöglicht vorteilhaft eine totraumfreie Abdichtung des Nebenkanalrohrs 11 direkt - oder im Bereich der Nebenkanalbohrung 8 - am Hauptkanal 6. Die schaltbare Anordnung lässt zudem zu, dass alle Nebenkanalräume in der geschlossenen Ventilstellung für Sterilisationsgase über einen mit offener (Berühr-)Schutzkappe 5 abgedeckten Nebenkanalkonnektor 12 und das Nebenkanalrohr 11 zugänglich sind. Gegenüber einem herkömmlichen Schalten des Nebenkanals zum Hauptkanal 6, etwa durch Öffnen oder Schließen einer Schlauchklemme, entsteht somit der Vorteil, dass sowohl die Sterilisation mittels Dampf oder Gas allgemein wie auch eine permanente Lagerung des Ports 100 jeweils (zumindest auch) bei geschlossener Ventilstellung bis zur Verwendung des Ports 100 möglich ist. Damit wird der Gefahr, dass die Zugabestelle oder das Zugabeventil, also die Verbindung von Nebenkanal zu Hauptkanal 6, zum Sterilisieren des Ports 100 geöffnet wird, dann aber bei oder vor Ingebrauchnahme des Ports 100 versehentlich nicht wieder verschlossenen wird, vorteilhaft verringert.

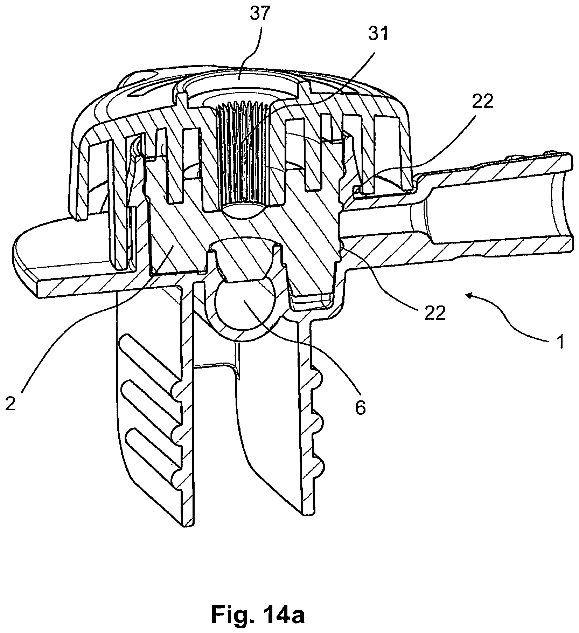

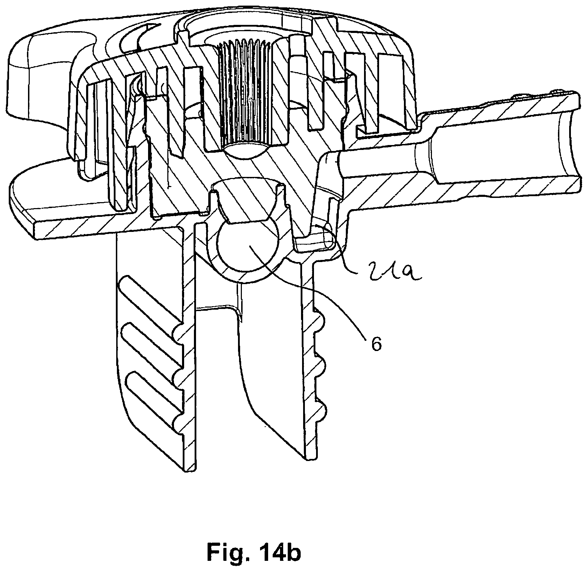

Die Fig. 14a und 14b zeigen einen Schnitt durch einen Port 100 einer weiteren erfindungsgemäßen Ausführungsform, mit einem bereits in Fig. 10 und 10a gezeigten Schalttopf 15. In dieser Ausführungsform verfügt der Port 100 im Schalttopf 15 über eine zusätzliche Abdichtungsmöglichkeit (auch als Zweitabdichtung oder Doppeldichtung bezeichnet) des Hauptkanals 6 gegenüber der Nebenkanalbohrung 8.

Das Dichtelement 2 weist in dieser Ausgestaltung, z. B. an seiner äußeren Mantelfläche, eine erhabene Radialdichtstruktur 22 auf, wie sie beispielsweise in einer umlaufenden oder geschlossenen oder ringförmigen Ausgestaltung in Fig. 13 gezeigt ist. Abweichend hiervon kann die Radialdichtstruktur 22 natürlich auch rund oder langlochförmig ausgebildet sein. Letzteres kann vorteilhaft dazu beitragen, eine Toleranz gegenüber nicht korrekt ausgeführten Schaltwinkeln zu erhöhen, oder umgekehrt, dass die Anforderungen an die Fertigungstoleranz gesenkt werden können.

Die Radialdichtstruktur 22 kann eine sich nach radial erhebende äußere Oberfläche aufweisen, welche der gebogenen, inneren Oberfläche in Umfangsrichtung der Zylinderform des Schalttopfs 15 nachempfunden ist oder dieser komplementär entspricht.

Von der Erfindung umfasst sind zudem Zwischen-Formgebungen, welche etwa hantelförmig ausgestaltet oder räumlich stärker gebogen sind als die gerade axiale Richtung und die zylindrische Umfangsrichtung des zylindrischen Schalttopfs 15.

Mittels der Radialdichtstruktur 22 kann unter Einbau-Vorspannung vorteilhaft eine dünne geschlossene oder ringförmige Anschmiege-Verpresszone um die Nebenkanalbohrung 8 im Schalttopf 15 herum entstehen. Hierdurch kann eine zusätzliche, zweite Abdichtung gegenüber dem Inneren des Nebenkanalrohrs 11 erzielt werden.

Vorteilhaft können hierbei die Ansprüche an die Toleranz der an der Dichtwirkung der oben beschriebenen ersten Dichtstrukturen beteiligten Bauteile gesenkt werden. Zugleich kann diese Ausgestaltung vorteilhaft die Dichtwirkung zu erhöhen.

Die Ausführung mit Zweitabdichtung oder Doppeldichtung, als zweifacher Abdichtung des Nebenkanals gegenüber dem Hauptkanal 6, weist in bestimmten erfindungsgemäßen Ausführungsformen Vorteile auf. Diese bestehen darin, dass eine oder beide der Abdichtungen vorwiegend oder vollständig axial verpresste Dichtungen sind, welche bei Erhöhung der Druckdifferenz zwischen den voneinander getrennten Fluidräumen in die eine Druckrichtung selbstverstärkend, in die andere Druckrichtung aber unter Senkung der Vorspannung wirksam sind. Es kann somit bei der jeweils ungünstigen Druckrichtung einen Grenzdruck geben, bei welchem die Vorspannung aufgehoben ist und die Dichtung abhebt. Beide Dichtungen sind nun aber bezüglich ihrer Wirkungsrichtung invers angeordnet. Es kann hierbei somit von einer bidirektionalen Dichtverstärkung gesprochen werden. Auf diese Weise kommt es bei wenigstens einer der Dichtungen zu einer Selbstverstärkung, wodurch sehr hohe Dichtgrenzdrücke bis zur Zerstörungsgrenze des Gehäuseelements erzielt werden können.

In manchen erfindungsgemäßen Ausführungsformen weist die Dichtzone an der Nebenkanalbohrung 8 zum Hauptkanal 6 eine Struktur auf, welche als Kapillare oder aufgrund von geringfügig nicht aufeinander abgestimmten oder übereinstimmenden Geometrien zwischen Gehäuseelement 1 und Dichtelement 2 eine undichte Stellung erzeugt wird. Gleichwohl besteht am Hauptkanal 6 kein Totraum bezogen auf die gewünschten Strömungsverhältnisse. Auf diese Weise kann man die besonders hohe Dichtwirkung der Nebenkanaldichtung am Schalttopf 15 mit der guten Gassterilisierbarkeit des Ports 100 und der guten Totraumfreiheit im Hauptkanal 6 kombinieren.

Da in vielen Fällen vor Beginn der Behandlung, z. B. der Dialyse, der Hauptkanal 6 gespült und befüllt wird, kann man hierzu verwendetes Fluid bereits in die Nebenfluidstrukturen eindringen und dort vorhandene Luftnester wenigstens teilweise auflösen oder ausspülen lassen, ohne dass das Ventil zu diesem Zweck geöffnet werden müsste.

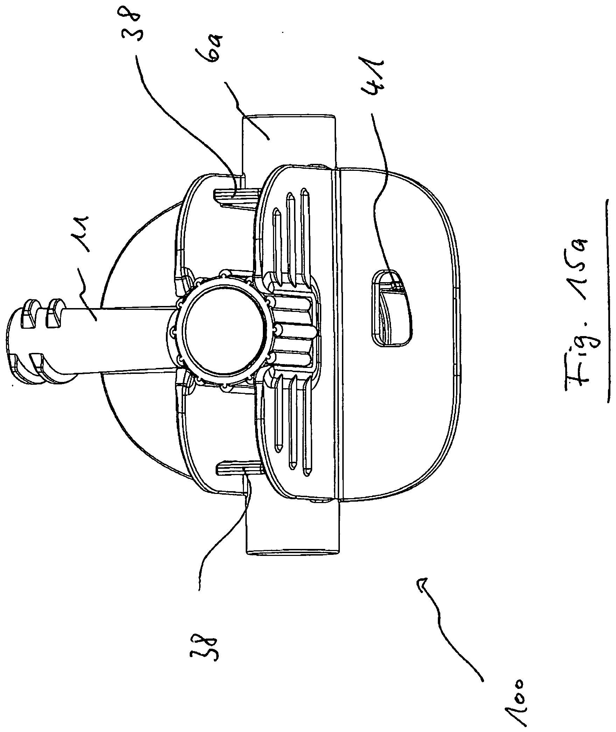

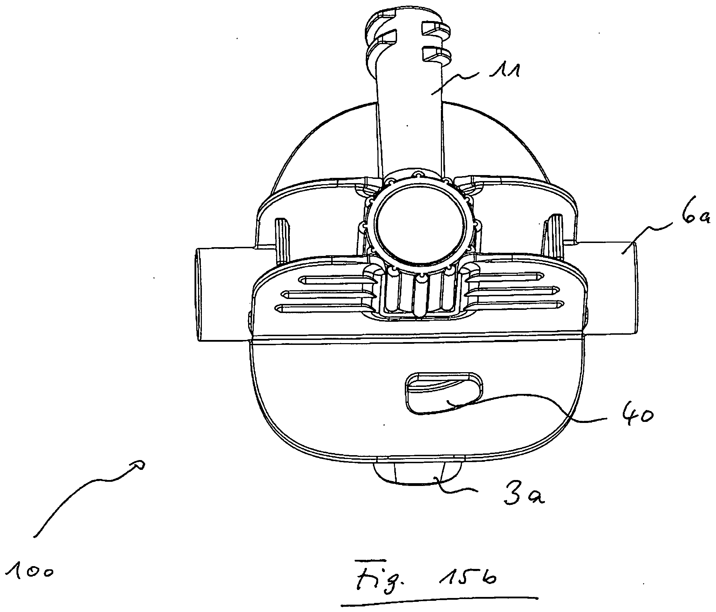

Die Fig. 15a und 15b zeigen eine weitere erfindungsgemäße Ausführungsform des Ports 100 mit einer Einrichtung zur Überwachung der Schaltstellung oder der Position des Betätigungselements 3. Die Einrichtung zur Überwachung der Schaltstellung interagiert mit taktilen und/oder optischen Sensoren am Maschinensockel. In der Stechschutzplatte 1a oder an einem anderen Abschnitt des Gehäuseelements 1 befindet sich vorzugsweise ein, beispielsweise im Wesentlichen langlochförmiger, Durchbruch 40, durch welchen ein Kontakt mit einer Schaltnocke 41 zu deren taktiler Überprüfung oder die optische Wahrnehmbarkeit der Schaltnocke 41 gewährleistet ist. Die Schaltnocke 41 kann derart im Maschinensockel angeordnet sein, dass sie für eine Überwachung mittels Sensoren zugänglich ist. Durch geeignete Ausgestaltung von Durchbruch 40, Schaltnocke 41, Maschinensockel und Sensorik kann zusätzlich ein Schlüssel-Schloss-Prinzip etabliert werden, wodurch nicht nur die Anwesenheit des Ports 100 in korrekter Position auf dem Maschinensockel erkannt wird, sondern zugleich eine Anwesenheits-Manipulation erschwert wird. Fig. 15a zeigt einen Zustand bei geschlossener Ventilstellung, Fig. 15b zeigt einen Zustand bei offener Ventilstellung.

In einigen erfindungsgemäßen Ausführungsformen, wie sie auch in den Figuren gezeigt sind, führt bereits eine geringe Drehung des Betätigungselements 3 zu einer beginnenden Drossel- oder Ventilwirkung des Ports 100. Daher sind in manchen erfindungsgemäßen Ausführungsformen zusätzlich zu den Drehrastungen Reibe- oder Feinrastzonen vorgesehen; in anderen erfindungsgemäßen Ausführungsformen ersetzen die Reibe- oder Feinrastzonen die Drehrastungen. Da aber die Drossel-Kennlinie, insbesondere bei ansonsten unveränderter Gestaltung der Nebenkanäle 9, 10 und der Dichtnase 21 des Dichtelements 2, nichtlinear verläuft, ist in bestimmten erfindungsgemäßen Ausführungsformen eine sich allmählich erweiternde keilförmige Dichtspalte vorgesehen, wie man sie auch bei Regelventilen findet. Bei dieser Ausgestaltung kann eine Linearität der Drosselwirkung über einen beliebig weiten Drehwinkelbereich, innerhalb welchem das Betätigungselement 3 gedreht werden kann, erzielt werden.

Veränderliche Drosselgrade oder Abdichtungsgrade bezogen auf die Nebenkanalbohrung 8 im Schalttopf 15 oder Septumtopf oder im Dichtelement 2 sind ferner dadurch erzielbar, dass beispielsweise keilförmig radial oder spiralförmig verlaufende Nebenkanalstrukturen im Dichtelement 2 vorgesehen werden. Diese bringen in Abhängigkeit vom eingestellten Drehwinkel verschieden große Querschnitte einer Fluidleitung innerhalb des Dichtelements 2 mit der Nebenkanalbohrung 8 in Verbindung.

In manchen erfindungsgemäßen Ausführungsformen ist ein Schutz vor einem Rückfluss des Fluids des Nebenkanals im Port 100 vorgesehen. Dieser Schutz verhindert ein Auslaufen von Flüssigkeit durch das Nebenkanalrohr 11, er erlaubt zugleich aber unter hinreichend geringem Druckabfall das Fördern von Nebenfluiden in den Hauptkanal 6 hinein. Dies kann mittels eines Rückschlagventils mit einer Zuström-Rückschlagventil-Funktion ausgeführt werden. Dabei steht das Rückschlagventil unter einer gewissen Vorspannung. Sie sorgt dafür, dass die Dichtwirkung ausreichend hoch ist, um zu verhindern, dass - bis zu einem bestimmten oder vorbestimmten Unterdruck im Hauptkanal 6 relativ zur Umgebung - Luft über das Nebenkanalrohr 11 eindringen kann.

Insbesondere die oben beschriebene, doppelt dichtende Ausführungsform des erfindungsgemäßen Ports 100 hat neben der offenen und der geschlossenen Ventilstellung bereits eine vorteilhafte Ventilrückschlagwirkung. Das Ventil verhindert zwar durch Selbstverstärkung das Austreten von Flüssigkeiten gegen hohe Druckdifferenzen, es bleibt aber aufgrund der Nachgiebigkeit und der Aufhebung der Vorspannung unter umgekehrtem Fluiddruck möglich, Flüssigkeiten nach Überwindung eines Druck-Grenzwertes in den Hauptkanal 6 einzubringen. Senkt man mit konstruktiven Maßnahmen die Vorspannung der Dichtung zur Nebenkanalbohrung 8 im Schalttopf 15, so erhält man ein Rückschlagventil mit beliebig gering einstellbarem Öffnungsdruck.

Ein Blockieren der entgegengesetzten Strömungsrichtung mittels des Rückschlagventils wird hierbei ebenso wie eine unerwünschte Drosselung erzielt.

Zur Verminderung der Vorspannung sind erfindungsgemäß verschiedene Möglichkeiten vorgesehen: Führt man die Radialdichtstruktur beispielsweise in einer Ringform aus, wobei der Ring einen - insbesondere deutlich - größeren Durchmesser als die Bohrung im Septumtopf hat, so erhöht man in Durchlassrichtung die druckbeaufschlagte Fläche deutlich und vermindert damit den Öffnungsdruck. Ebenso ist es möglich, das Dichtelement 2 in der Rückschlagventilstellung radial weniger eigensteif zu gestalten und die Gegenabstützung durch die Ringstege im Betätigungselement 3 mittels Unterbrechungen und/oder Nuten herabzusetzen.

Mittels der letztgenannten Ausgestaltung erlaubt der erfindungsgemäße Port 100 neben den bisher diskutierten Stellungen (offene Ventilstellung und geschlossene Ventilstellung) auch noch eine Zufluss-Rückschlag-Stellung. Man kann mittels des erfindungsgemäßen Ports 100 in einigen Ausführungsformen somit sowohl Ventile verwirklichen mit entweder nur zwei Schaltstellungen, oder mit drei Raststellungen und drei verschiedenen Fluidfunktionen. In der geschlossenen Ventilstellung bleibt der Vorteil der redundanten und/oder totraumfreien Abdichtung an der Nebenkanalbohrung 8 gegenüber dem Hauptkanal 6 stets erhalten.

**Bezugszeichenliste**

| **Bezugszeichen** | **Beschreibung** |
|---|---|
| 1 | Gehäuseelement |
| 1a | Stechschutzplatte |
| 2 | Dichtelement |
| 2a | Abschnitt des Dichtelements |
| 3 | Betätigungselement |
| 3a, b | Knebel |
| 3c | Pfeil |
| 4 | Anschlussschläuche |
| 5 | Schutzkappe |
| 6 | Hauptkanal |
| 6a | Schlauchmuffen |
| 7 | Septumbohrung oder Septummündung |
| 7a | Septum |
| 8 | Nebenkanalbohrung oder Nebenkanalmündung |
| 9 | Nebenkanal im Gehäuseelement |
| 10 | Nebenkanal im Dichtelement |
| 11 | Nebenkanalrohr |
| 12 | Nebenkanalkonnektor |
| 13 | Hauptkanalströmung |
| 14 | Nebenkanalströmung |
| 15 | Schalttopf |
| 16a, b | Nebenkanalbohrung im Schalttopf (erste bzw. zweite Ausführung) |
| 17 | abgeplattete Innenform des Hauptkanals |
| 17a | Abplattung des Dichtelements |
| 18 | Verschlussstirn eines Septum-Dichtelements |
| 19 | Formwechselkante des Hauptkanals |
| 20 | stirnseitige Abdichtfläche des Dichtelements |
| 21 | Dichtnase des Dichtelements zum Hauptkanal |
| 21a | Vertiefung |
| 22 | Radialdichtstruktur des Dichtelements zur Nebenkanalbohrung im Schalttopf |
| 23 | Stopfbuchsdichtring des Dichtelements |
| 24 | Stopfbuchsdichtzone des Schalttopfs |
| 25 | Sterilisations-, Stütz- und Kompressibilitätsstege und -nuten im Dichtelement |
| 26 | Sterilisations-, Stütz- und Kompressibilitätsstege und -nuten im Betätigungselement |
| 27 | Drehmitnehmer |
| 28 | Gestufter Bereich des Schalttopfs |
| 29 | Axialstützrippe des Betätigungselements |
| 30 | Bombierte Formen im Septum-Dichtbereich |
| 31 | Einstichkanal |
| 32a, b | Rastelemente |
| 33 | Drehrastung |
| 33a | Rastnase |
| 33b | Rastsenke |
| 34 | Drehanschläge |
| 35 | Bogenrippen |
| 36 | Rillenstruktur im Einstechkanal |
| 37 | Berührschutzsteg um die Einstichmündung |
| 38 | Schnappzungen zur Fixierung an der Behandlungsmaschine |
| 39 | Piktogramme zur Funktionsvisualisierung |
| 39a | Crescendo-Symbol |
| 40 | Schaltstellungs-Durchbruch |
| 41 | Schaltstellungs-Nocke |
| 42 | Kanüle |

## Patentansprüche

1. Medizinischer Port (100), wobei der Port (100) einen Hauptkanal (6) mit einem Lumen zum Leiten eines ersten Fluids durch den Port (100) hindurch aufweist, mit einer Nebenkanalmündung (8) eines Nebenkanals zum Zugeben eines zweiten Fluids in den Hauptkanal (6),
wobei der Port (100) wenigstens ein Gehäuseelement (1) und wenigstens ein relativ zum Gehäuseelement (1) aus einer ersten Position in eine zweite Position überführbar angeordnetes Betätigungselement (3) aufweist,
wobei der Port (100) einen Dichtabschnitt aufweist, welcher angeordnet ist, um bei Überführen des Betätigungselements (3) von einer Position in die andere zwischen einer ersten Stellung des Dichtabschnitts, in welcher der Dichtabschnitt die Nebenkanalmündung (8) nicht verschließt oder bedeckt, sprich einer offenen Stellung, und einer zweiten Stellung des Dichtabschnitts, in welcher der Dichtabschnitt die Nebenkanalmündung (8) verschließt oder bedeckt, sprich einer geschlossenen Stellung, verdrehbar zu sein, **dadurch gekennzeichnet, dass**
der Dichtabschnitt eine Dichtnase (21) aufweist, welche in axialer Richtung des Dichtabschnitts über diesen vorsteht,
wobei die Dichtnase (21) angeordnet ist, um bei einer Drehung des Betätigungselements (3), oder beim Überführen des Betätigungselements (3) von einer Position in die andere, auf einer Drehkurve von einer ersten Stellung in eine zweite Stellung auf der Drehkurve bewegt zu werden, wobei die Dichtnase (21) in der ersten Stellung die Nebenkanalmündung (8) nicht verschließt oder bedeckt, und
wobei die Dichtnase (21) die Nebenkanalmündung (8) in der zweiten Stellung verschließt oder bedeckt.

2. Port nach Anspruch 1, wobei der Dichtabschnitt angeordnet und ausgestaltet ist, um eine Durchgängigkeit des Hauptkanals (6) für das erste Fluid weder in seiner ersten Stellung noch in seiner zweiten Stellung zu beeinträchtigen.

3. Port nach einem der vorangegangenen Ansprüche, wobei kein Abschnitt des Dichtabschnitts im Lumen des Hauptkanals (6) vorliegt.

4. Port nach einem der Ansprüche 1 bis 3, wobei der Hauptkanal (6) zusätzlich zur Nebenkanalmündung (8) eine Septummündung (7) aufweist, welche im Gebrauch des Ports (100) mit einem mittels Kanüle (42) durchstechbaren Septum (7a) verschlossen ist.

5. Port nach einem der vorangegangenen Ansprüche,
wobei der Dichtabschnitt eine stirnseitige Abdichtfläche (20) aufweist, welche angeordnet ist, um bei einer Drehung des Dichtabschnitts entlang einer Drehkurve von einer ersten Stellung in eine zweite Stellung auf der Drehkurve bewegt zu werden,
wobei die Abdichtfläche (20) in der zweiten Stellung die Nebenkanalmündung (8) verschließt oder bedeckt, wobei die Abdichtfläche (20) die Nebenkanalmündung (8) in der ersten Stellung nicht verschließt oder bedeckt,
und
wobei sich die Abdichtfläche (20) parallel zu einer Hauptquerschnittsebene und/oder senkrecht zu einer Drehachse des Dichtabschnitts erstreckt.

6. Port nach einem der vorangegangenen Ansprüche ,
wobei das Gehäuseelement (1) wenigstens einen Abschnitt aufweist,
welcher eine Vertiefung (21a) zur Aufnahme der hierin bewegbaren Dichtnase (21) aufweist, und
welcher die Nebenkanalmündung (8) aufweist oder hieran angrenzt.

7. Port nach einem der vorangegangenen Ansprüche,
wobei die Dichtnase (21) eine sowohl zu einer Stirnseite der Dichtnase (21) als auch zu einer lateralen Seitenfläche oder Umfangsfläche des Dichtabschnitts offene Nut aufweist.

8. Port nach Anspruch 7,
wobei die Nut in der ersten Stellung des Dichtabschnitts derart an einer Öffnung eines Nebenkanalrohrs (11) anliegt, dass sie den Fluidweg des Nebenkanalrohrs (11) über den Dichtabschnitt hinweg fortsetzt, und wobei die Nut in der zweiten Stellung des Dichtabschnitts nicht mit dem Nebenkanalrohr (11) oder dessen Öffnung in Fluidverbindung steht.

9. Port nach einem der Ansprüche 5 bis 8,
wobei der Dichtabschnitt zusätzlich zur stirnseitigen Abdichtfläche (20) oder zusätzlich zur Dichtnase (21) eine erhabene, geschlossene Dichtstruktur (22) aufweist, welche in der zweiten Stellung des Dichtabschnitts die Öffnung des Nebenkanalrohrs (11) verschließt oder einen Austritt von Fluid aus der Öffnung verhindert.

10. Port nach einem der vorangegangenen Ansprüche, wobei der Dichtabschnitt als ein separates Dichtelement (2) ausgestaltet ist.

11. Port nach einem der vorangegangenen Ansprüche, wobei sowohl die Septummündung (7) als auch die Nebenkanalbohrung (8) gemeinsam in einer Querschnittshälfte des Hauptkanals (6) angeordnet sind.

12. Blutschlauch zur Verwendung bei einer extrakorporalen Blutbehandlung, wobei der Blutschlauch wenigstens einen Port (100) nach einem der vorangegangenen Ansprüche aufweist.

13. Medizinische Behandlungsvorrichtung, welche mit wenigstens einem Blutschlauch gemäß Anspruch 12 verbunden ist.

14. Medizinische Behandlungsvorrichtung nach Anspruch 13 welche eine Aufnahme zum Aufnehmen oder Befestigen des Ports gemäß einem der Ansprüche 1 bis 11 aufweist und/oder welche eine Einrichtung zum Erkennen der Anwesenheit des Ports gemäß einem der Ansprüche 1 bis 11 und/oder zum Erkennen von dessen Ventilsstellung aufweist.

15. Medizinische Behandlungsvorrichtung nach einem der Ansprüche 13 bis 14, welche als Blutbehandlungsvorrichtung, insbesondere als Vorrichtung zur Apherese oder Dialyse, wiederum insbesondere zur Hämodialyse, Hämofiltration, Hämodiafiltration, ausgestaltet ist.

## Claims

1. A medical port (100), wherein the port (100) comprises a main channel (6) with a lumen for leading a first fluid through the port (100), having a secondary channel mouth (8) of a secondary channel for adding a second fluid into the main channel (6),
wherein the port (100) comprises at least one housing element (1) and at least one actuating element (3) arranged such that it can be transferred relative to the housing element (1) from a first position to a second position,
wherein the port (100) comprises a sealing portion arranged to be rotatable when transferring the actuating element (3) from a position to the other between
a first position of the sealing portion in which the sealing portion doesn't close or cover the secondary channel mouth (8), in other words an open position,
and a second position of the sealing portion in which the sealing portion closes or covers the secondary channel mouth (8), in other words a closed position.
**characterized in that**,
the sealing portion comprises a sealing lug (21) which projects beyond the sealing portion in the axial direction of the latter,
wherein the sealing lug (21) is arranged to be moved on a rotation curve from a first position to a second position on the rotation curve when the actuating element (3) is rotated, or when the actuating element (3) is transferring from a position to the other,
wherein the sealing lug (21) doesn't close or cover the secondary channel mouth (8) in the first position, and wherein the sealing lug (21) closes or covers the secondary channel mouth (8) in the second position.

2. The port according to claim 1, wherein the sealing portion is arranged and configured so as not to interfere with a continuity of the main channel (6) for the first fluid neither in its first position nor in its second position.

3. The port according to anyone of the preceding claims, wherein no portion of the sealing portion is present in the lumen of the main channel (6).

4. The port according to anyone of claims 1 to 3, wherein the main channel (6), in addition to the secondary channel mouth (8), comprises a septum mouth (7) which, when using the port (100), is closed with a septum (7a) which can be perforated by means of a cannula (42).

5. The port according to anyone of the preceding claims,
wherein the sealing portion comprises a sealing surface (20) at the front side arranged to be moved from a first position to a second position on the rotation curve when rotating the sealing portion along a rotation curve.
wherein in the second position the sealing surface (20) closes or covers the secondary channel mouth (8),
wherein the sealing surface (20) doesn't close or cover the secondary channel mouth (8) in the first position, and
wherein the sealing surface (20) extends parallel to a main cross-sectional plane and/or perpendicular to a rotation axis of the sealing portion.

6. The port according to anyone of the preceding claims,
wherein the housing element (1) comprises at least one portion,
which comprises a cavity (21a) for receiving the movable sealing lug (21) therein, and
which comprises or adjoins to the secondary channel mouth (8) .

7. The port according to anyone of the preceding claims, wherein the sealing lug (21) comprises an open groove to a front side of the sealing lug (21) as well as to a lateral side or peripheral surface of the sealing portion.

8. The port according to claim 7,
wherein the groove in the first position of the sealing portion abuts against an opening of a secondary channel pipe (11) such that it continues the fluid path of the secondary channel pipe (11) across the sealing portion, and
wherein the groove in the second position of the sealing portion is not in fluid communication with the secondary channel pipe (11) or its opening.

9. The port according to anyone of claims 5 to 8,
wherein the sealing portion, in addition to the sealing surface (20) at the front side or in addition to the sealing lug (21), comprises a raised closed sealing structure (22) which, in the second position of the sealing portion, closes the opening of the secondary channel pipe (11) or prevents fluid from escaping through the opening.

10. The port according to anyone of the preceding claims,
wherein the sealing portion is configured as a separate sealing element (2).

11. The port according to anyone of the preceding claims,
wherein the septum mouth (7) as well as the secondary channel bore (8) are arranged together in a cross-sectional half of the main channel (6).

12. A blood tube for use in an βextracorporeal blood treatment,
wherein the blood tube comprises at least one port (100) according to anyone of the preceding claims.

13. A medical treatment apparatus connected to at least one blood tube according to claim 12.

14. The medical treatment apparatus according to claim 13, which comprises a receptacle for receiving or attaching the port according to anyone of claims 1 to 11 and/or which comprises a device for detecting the presence of the port according to anyone of claims 1 to 11 and/or for detecting the valve position thereof.

15. The medical treatment apparatus according to anyone of claims 13 to 14 designed as a blood treatment apparatus, in particular as an apparatus for apheresis or dialysis, more particularly for hemodialysis, hemofiltration, hemodiafiltration.

## Revendications

1. Un port médical (100), le port (100) comprenant un conduit principal (6) avec une cavité pour diriger un premier fluide à travers le port (100), ayant une embouchure de conduit secondaire (8) d'un conduit secondaire pour ajouter un second fluide dans le conduit principal (6),
où le port (100) comprend au moins un élément de boîtier (1) et au moins un élément d'actionnement (3) agencé par rapport à l'élément de boîtier (1) de manière à pouvoir être transféré d'une première position à une seconde position,
où le port (100) comprend une section d'étanchéité agencée de façon à pouvoir pivoter, lors du transfert de l'élément d'actionnement (3) d'une position à l'autre, entre une première position de la section d'étanchéité dans laquelle la section d'étanchéité ne ferme pas ou ne recouvre pas l'embouchure du conduit secondaire (8), c'est-à-dire une position ouverte,
et une seconde position de la section d'étanchéité dans laquelle la section d'étanchéité ferme ou recouvre l'embouchure du conduit secondaire (8), c'est-à-dire une position fermée,
**caractérisé en ce que**,
la section d'étanchéité comprend une patte d'étanchéité (21) qui dépasse de la section d'étanchéité dans une direction axiale de cette dernière,
où la patte d'étanchéité (21) est agencée de façon à être déplacée, lors d'une rotation de l'élément d'actionnement (3) ou lors du transfert de l'élément d'actionnement (3) d'une position à l'autre sur une courbe de rotation, d'une première position à une seconde position sur la courbe de rotation.
où la patte d'étanchéité (21) ne ferme pas ou ne recouvre pas l'embouchure du conduit secondaire (8) dans la première position, et
où la patte d'étanchéité (21) ferme ou recouvre l'embouchure du conduit secondaire (8) dans la seconde position.

2. Le port selon la première revendication, où la section d'étanchéité est agencée et conçue de façon à ne pas interférer avec une continuité du conduit principal (6) pour le premier fluide, ni dans sa première position, ni dans sa seconde position.

3. Le port selon l'une quelconque des revendications précédentes, où aucune section de la section d'étanchéité n'est présente dans la cavité du conduit principal (6).

4. Le port selon l'une quelconque des revendications 1 à 3, où le conduit principal (6) comprend, en plus de l'embouchure du conduit secondaire (8), une embouchure de septum (7) qui, lors de l'utilisation du port (100), est fermée avec un septum (7a) perforable au moyen d'une canule (42).

5. Le port selon l'une quelconque des revendications précédentes,
où la section d'étanchéité comprend une surface d'étanchéité (20) côté frontal agencée de façon à être déplacée d'une première position à une seconde position sur la courbe de rotation lors de la rotation de la section d'étanchéité le long d'une courbe de rotation.
où, dans la seconde position, la surface d'étanchéité (20) ferme ou recouvre l'embouchure du conduit secondaire (8),
où la surface d'étanchéité (20) ne ferme pas ou ne recouvre pas l'embouchure du conduit secondaire (8) dans la première position, et
où la surface d'étanchéité (20) s'étend parallèlement à un plan de coupe transversale principal et/ou perpendiculairement à un axe de rotation de la section d'étanchéité.

6. Le port selon l'une quelconque des revendications précédentes,
où l'élément de boîtier (1) comprend au moins une section qui comprend une cavité (21a) pour y recevoir la patte d'étanchéité (21) mobile, et
qui comprend l'embouchure du conduit secondaire (8) ou la jouxte.

7. Le port selon l'une quelconque des revendications précédentes,
où la patte d'étanchéité (21) comprend une rainure ouverte sur une face frontale de la patte d'étanchéité (21) ainsi que sur une surface latérale ou périphérique de la section d'étanchéité.

8. Le port selon la revendication 7,
où la rainure dans la première position de la section d'étanchéité se situe contre un orifice d'un tuyau de conduit secondaire (11) de sorte qu'elle poursuit le trajet fluidique du tuyau de conduit secondaire (11) à travers la section d'étanchéité, et
où la rainure dans la seconde position de la section d'étanchéité n'est pas en communication fluidique avec le tuyau de conduit secondaire (11) ou son orifice.

9. Le port selon l'une quelconque des revendications 5 à 8, où la section d'étanchéité, en plus de la surface d'étanchéité (20) côté frontal, ou en plus de la patte d'étanchéité (21), comprend une structure d'étanchéité (22) fermée en relief qui, dans la seconde position de la section d'étanchéité, ferme l'orifice du tuyau de conduit secondaire (11) ou empêche le fluide de s'échapper par l'orifice.

10. Le port selon l'une quelconque des revendications précédentes, où la section d'étanchéité est conçue comme un élément d'étanchéité séparé (2).

11. Le port selon l'une quelconque des revendications précédentes, où à la fois l'embouchure de septum (7) ainsi que l'alésage du conduit secondaire (8) sont agencés ensemble dans une moitié de coupe transversale du conduit principal (6).

12. Un tuyau sanguin destiné à être utilisé dans un traitement sanguin extracorporel, où le tuyau sanguin comprend au moins un port (100) selon l'une quelconque des revendications précédentes.

13. Un appareil de traitement médical relié à au moins un tuyau sanguin selon la revendication 12.

14. L'appareil de traitement médical selon la revendication 13 comprenant un réceptacle pour recevoir ou fixer le port selon l'une quelconque des revendications 1 à 11 et/ou comprenant un dispositif pour détecter la présence du port selon l'une quelconque des revendications 1 à 11 et/ou pour détecter la position de la vanne de celui-ci.

15. L'appareil de traitement médical selon l'une quelconque des revendications 13 à 14 conçu comme un appareil de traitement du sang, en particulier comme un appareil d'aphérèse ou de dialyse, plus particulièrement, pour l'hémodialyse, l'hémofiltration, l'hémodiafiltration.
